# EUROPÄISCHE PATENTSCHRIFT

(11) **EP 4 024 489 B1**
(45) Veröffentlichungstag und Bekanntmachung des Hinweises auf die Patenterteilung: **04.10.2023**
(21) Anmeldenummer: 21218393.3
(22) Anmeldetag: 30.12.2021
(51) Int. Cl.: H10K 85/60, H10K 30/30, H10K 30/50

(54) **VERBINDUNG UND DEREN VERWENDUNG IN ORGANISCHEN ELEKTRONISCHEN BAUELEMENTEN**
COMPOUND AND ITS USE IN ORGANIC ELECTRONIC COMPONENTS
RACCORDEMENT ET SON UTILISATION DANS LES COMPOSANTS ÉLECTRONIQUES ORGANIQUES

(30) Priorität: 31.12.2020 DE 102020135172
(43) Veröffentlichungstag der Anmeldung: 06.07.2022
(73) Patentinhaber: Heliatek GmbH, 01139 Dresden (DE)
(72) Erfinder: Gerdes, Olga, 89077 Ulm (DE); Grimm, Nina, 89077 Ulm (DE); Hildebrandt, Dirk, 89077 Ulm (DE)

(56) Entgegenhaltungen:
- EP-A1- 3 188 270
- WO-A1-2011/161262
- CN-A- 104 086 561
- DE-A1-102012 104 247
- DE-A1-102013 101 712
- DE-A1-102013 101 713

## Beschreibung

Die Erfindung betrifft eine Verbindung der allgemeinen Formel(I), eine Verwendung einer solchen Verbindung in einem organischen elektronischen Bauelement, sowie ein organisches elektronisches Bauelement mit einer solchen Verbindung.

Elektronische Bauelemente aus organischen Materialien sind für die Anwendung als LEDs (OLED) und organischen photovoltaischen Elementen (OPV), also organischen Solarzellen, bekannt. Die verwendeten organischen Materialien erfüllen in diesen organischen elektronischen Bauelementen unterschiedliche Funktionen, insbesondere einen Ladungstransport, eine Lichtemission oder Lichtabsorption. Organische Materialien in optoelektronischen Bauelementen können dabei Polymere oder kleine Moleküle sein und in Lösung oder Emulsion durch nasschemische Prozesse wie Coaten oder Drucken oder im Vakuum durch Sublimation zu dünnen Schichten verarbeitet werden. Organisch elektronische Bauelemente sind beispielsweise Displays, Datenspeicher oder Transistoren, aber auch organische optoelektronische Bauelemente, insbesondere Solarzellen oder Photodetektoren. Solarzellen oder Photodetektoren weisen eine photoaktive Schicht auf, in der bei Einfall von elektromagnetischer Strahlung gebundene Elektronen-Loch-Paare (Exzitonen) als Ladungsträger erzeugt werden. Die Exzitonen gelangen durch Diffusion an eine Grenzfläche, an der Elektronen und Löcher voneinander getrennt werden. Das Material, welches die Elektronen aufnimmt, wird als Akzeptor, und das Material, welches die Löcher aufnimmt, wird als Donor bezeichnet. Weitere organische elektronische Bauelemente sind Licht-emittierende Bauelemente, die Licht aussenden, wenn sie von Strom durchflossen werden. Organische elektronische Bauelemente umfassen mindestens zwei Elektroden, wobei eine Elektrode auf einem Substrat aufgebracht ist und die andere als Gegenelektrode fungiert. Zwischen den Elektroden befindet sich mindestens eine photoaktive Schicht, vorzugsweise eine organische photoaktive Schicht. Weitere Schichten, beispielsweise Transportschichten, können zwischen den Elektroden angeordnet sein.

Es werden nach wie vor organische halbleitende Materialien gesucht, die bei Verwendung in organischen elektronischen Bauelementen zu einer Verbesserung der Eigenschaften der Bauelemente führen. Das Absorptionsspektrum von bekannten Absorbern deckt den blauen Spektralbereich nicht vollständig ab. Zur Erhöhung des Absorptionsbereichs und damit zur Steigerung der Effizienz von Solarzellen werden deshalb Absorber benötigt, die im Spektralbereich zwischen 400 nm und 600 nm, insbesondere zwischen 480 und 580 nm, stark absorbieren und eine Spannung im Bereich von 1 V aufweisen. Dies insbesondere ist für die Verwendung in Tandem- und Triplet-Zellen vorteilhaft.

Aus dem Stand der Technik sind organische Verbindungen für elektrische Bauelemente bekannt. US20190043926A1 offenbart organische Verbindungen für photoelektrische Wandlerelemente zum Umwandeln von Strom in Strahlung. Derartige Verbindungen sind beim Verdampfen im Vakuum nicht stabil, und können deshalb nur aus Flüssigkeit prozessiert werden. Akzeptor-Donor-Akzeptor Verbindungen mit Thienothiophen-Einheiten sind aus DE 10 2012 104247 A1, DE 10 2013 101713 A1 und WO 2011/161262 A1 bekannt.

Nachteilig aus dem Stand der Technik ist insbesondere, dass die Spannung vieler Absorbermaterialien in einem Absorptionsbereich zu gering ist, insbesondere im Bereich von weit unter 1,0 V, insbesondere im Bereich von 0,9 V bis 0,95 V liegt. Bei Mehrfachzellen ist allerdings die Zelle mit der geringsten Spannung der limitierende Faktor für das gesamte elektrische Bauelement.

Die Aufgabe wird durch die Gegenstände der unabhängigen Ansprüche gelöst. Vorteilhafte Ausgestaltungen ergeben sich aus den Unteransprüchen.

Die Aufgabe wird insbesondere gelöst durch die Bereitstellung einer Verbindung der allgemeinen Formel I

A1-M-(T1)ₐ-(T2)_{b}-(T3)_{c}-A2 (I)

mit den Parametern a, b, c jeweils unabhängig voneinander 0, 1 oder 2, mit der Maßgabe, dass mindestens einer der Parameter a, b, c = 1 oder 2 ist,
mit der Gruppe M ausgewählt aus der Gruppe bestehend aus: 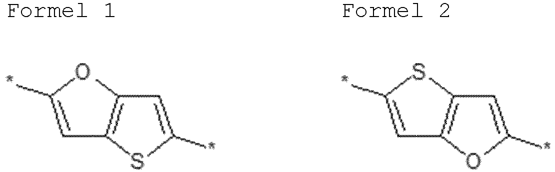 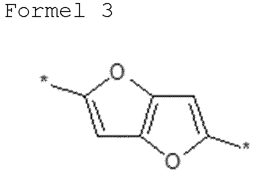
wobei * die Anknüpfung an die Gruppen T1, T2, T3, A1 und A2 bezeichnet, wobei H-Atome der Formeln 1 bis 4 durch Alkyl, O-Alkyl, S-Alkyl, oder Halogen substituiert sein können;
mit den Gruppen T1, T2 und T3 unabhängig voneinander ausgewählt aus der Gruppe bestehend aus: 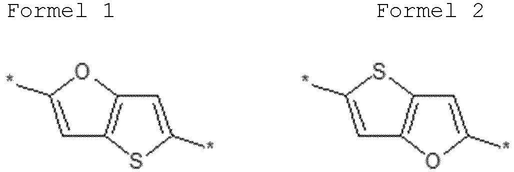 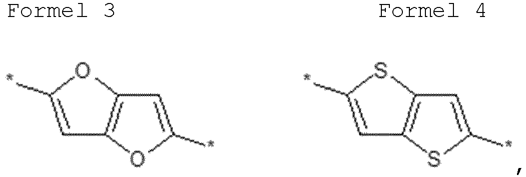
wobei H-Atome der Formeln 1 bis 4 durch Alkyl, O-Alkyl, S-Alkyl, oder Halogen substituiert sein können, 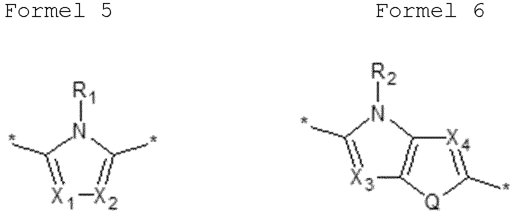 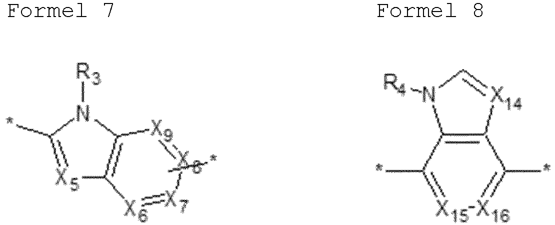
mit R1 bis R4 jeweils unabhängig voneinander ausgewählt aus der Gruppe bestehend aus H, Alkyl, Alkenyl, Aryl und Heteroaryl; 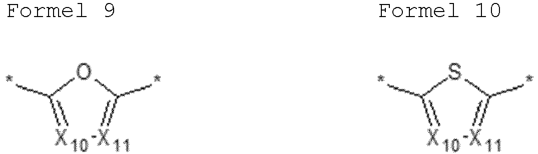 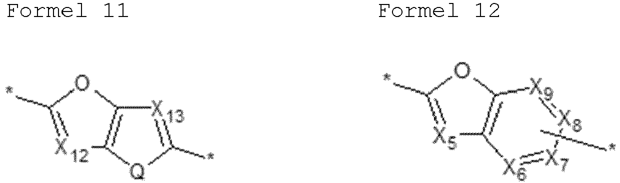 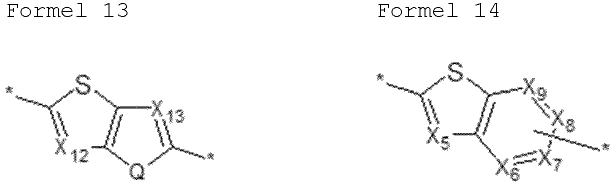 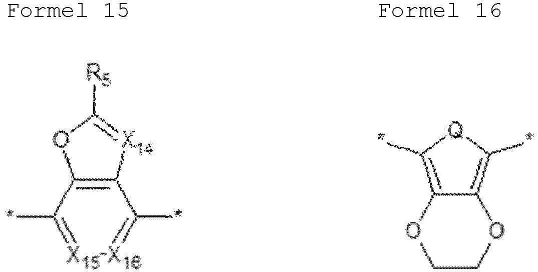 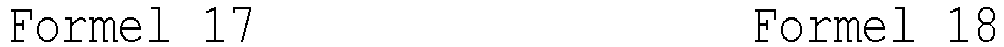 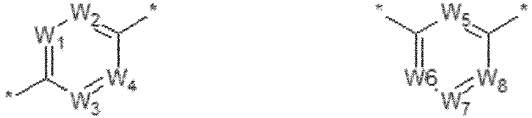
mit X₁ bis X₁₆ unabhängig voneinander ausgewählt aus N oder CR₆, mit der Maßgabe, dass in den Formeln 7, 12 und 14 jeweils eine Gruppe aus X₆ bis X₉ die Anknüpfung * an eine weitere Gruppe in der Verbindung der allgemeinen Formel I bezeichnet,
mit Q ausgewählt aus der Gruppe bestehend aus O, S, und NR₇,
mit W₁ bis W₈ jeweils unabhängig voneinander ausgewählt aus N und CR₈, wobei R₆, R₇ und R₈ jeweils unabhängig voneinander ausgewählt sind aus der Gruppe bestehend aus H, CN, Alkyl, Alkenyl, O-Alkyl, S-Alkyl, Aryl, und Heteroaryl, wobei H-Atome von Alkyl, Alkenyl, O-Alkyl, S-Alkyl, Aryl, und Heteroaryl substituiert sein können;
wobei die elektronenziehenden Gruppen A1 und A2 unabhängig voneinander elektronenziehende Gruppen mit zumindest einer C=C-Doppelbindung sind, und wobei * die Anknüpfung an eine weitere Gruppe M, T1, T2, T3, und A2 in der Verbindung der allgemeinen Formel I bezeichnet.

Das konjugierte n-Elektronensystem des Donorbereichs (M-(T1)ₐ-(T2)_{b}-(T3)_{c}) der Verbindungen der allgemeinen Formel I kann über den Donorblock M mit den elektronenziehenden Gruppen A1 und A2 hinaus erweitert werden, indem zumindest ein weiterer Donorblock T1, T2 und/oder T3 eingebaut wird. Erfindungsgemäß ist insbesondere der Baustein mit einem anellierten Furan-Thiophen, Thiophen-Furan, Furan-Furan, und/oder Thiophen-Thiophen Baustein:

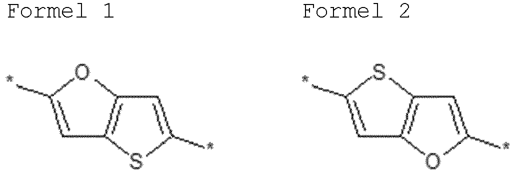

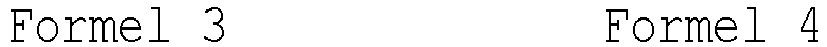

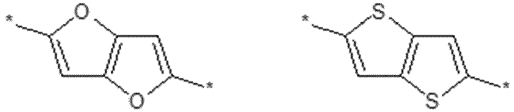

Wichtig, insbesondere zur Erhöhung der Aromatiziät der Verbindungen. Der Baustein führt insbesondere zu einer Absenkung des HOMO innerhalb der Verbindung, wodurch die Verbindung in einem elektronischen Bauelement mehr Spannung liefert. Aufgrund des strukturellen Merkmales der Gruppe M weisen die vorliegenden Verbindungen eine besonders hohe optische Dichte, bevorzugt im sichtbaren Spektralbereich auf, insbesondere eine hohes Integral über die optische Dichte im Absorptionsspektrum im Vergleich zu nicht erfindungsgemäßen Verbindungen, die das oben beschriebene Strukturelement nicht aufweisen. Unter "Integral" wird dabei der Flächeninhalt unterhalb einer Kurve im Absorptionsspektrum verstanden, das ein wichtiges Merkmal für die Eignung des Materials als photosensitives Material ist.

Unter einem Heteroatom werden gemäß der vorliegenden Erfindung insbesondere O, S, oder N verstanden.

Unter substituiert bzw. einem Substituenten wird im Sinne der vorliegenden Erfindung insbesondere der Austausch eines oder mehrerer H-Atome gegen eine andere Gruppe oder ein anderes Atom verstanden. Ein Substituent ist insbesondere ein Halogen oder ein Pseudohalogen, bevorzugt F, Cl oder CN, eine Alkyl-Gruppe, eine Alkenyl-Gruppe oder eine Aryl-Gruppe.

In einer besonders bevorzugten Ausführungsform der Erfindung ist b=0.

In einer besonders bevorzugten Ausführungsform der Erfindung ist sind T1 und T3 jeweils unabhängig voneinander ausgewählt aus den Formeln 1 bis 4.

Die erfindungsgemäßen chemischen Verbindungen der allgemeinen Formel I weisen Vorteile im Vergleich zum Stand der Technik auf. Vorteilhafterweise können verbesserte Absorber für organische elektronische Bauelemente, insbesondere photovoltaische Elemente, bereitgestellt werden. Vorteilhafterweise werden Absorbermaterialien für den roten und nah-infraroten Spektralbereich mit einer hohen Absorptionsstärke bereitgestellt. Vorteilhafterweise weisen die Absorber eine erhöhte Photospannung auf im Vergleich zu anderen ADA Absorbern ohne einen anellierten Furan-Thiophen, Thiophen-Furan, Furan-Furan, und/oder Thiophen-Thiophen Baustein (Formel 1 bis 4). Vorteilhafterweise ist der Leerlaufspannung Uoc elektronischer Bauelement mit einer erfindungsgemäßen Verbindung erhöht. Vorteilhafterweise absorbieren die erfindungsgemäßen Verbindungen im Spektralbereich zwischen 400 nm und 600 nm, insbesondere zwischen 480 und 580 nm, besonders stark und liefern eine Spannung in einem elektronischen Bauelement im Bereich von 1 V. Vorteilhafterweise kann die Effizienz von photovoltaischen Elementen erhöht werden. Vorteilhafterweise sind die erfindungsgemäßen Verbindungen weitgehend rückstandsfrei im Vakuum verdampfbar, und sind damit für eine Vakuumprozessierung zur Herstellung von photovoltaischen Elementen geeignet. Dies ist insbesondere vorteilhaft bei Mehrfachzellen, bevorzugt Tandem- oder Triplett-Zellen, oder Mischschichten, da die kleinste Spannung der Schicht limitierend für die Gesamtspannung der Zelle ist.

Die erfindungsgemäßen Verbindungen sind insbesondere sogenannte "kleine Moleküle", wobei darunter nicht-polymere oligomere organische Moleküle mit einer molaren Masse zwischen 100 bis 2000 g/mol verstanden werden.

Gemäß einer Weiterbildung der Erfindung ist vorgesehen, dass T1 unabhängig voneinander ausgewählt ist aus der Gruppe bestehend aus: 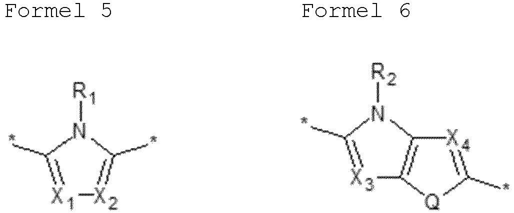 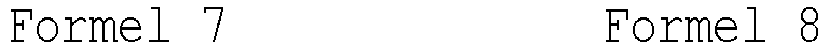 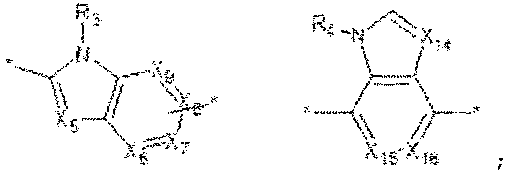 wobei bevorzugt X₁ und X₂ und/oder X₃ und X₄ CR₆ sind, bevorzugt ist R₆ ausgewählt aus H und Alkyl; und/oder wobei bevorzugt X₅ CR₆ ist mit R₆ H oder Alkyl, und/oder wobei bevorzugt X₆ bis X₉ CR₆ sind; und/oder wobei bevorzugt R₁ bis R₃ unabhängig voneinander H, Alkyl oder Aryl sind; und/oder wobei bevorzugt R₄ H oder Alkyl ist.

In einer bevorzugten Ausführungsform der Erfindung sind X₁ bis X₁₆ CR₆.

In einer bevorzugten Ausführungsform der Erfindung ist T1 ausgewählt aus der Gruppe bestehend aus 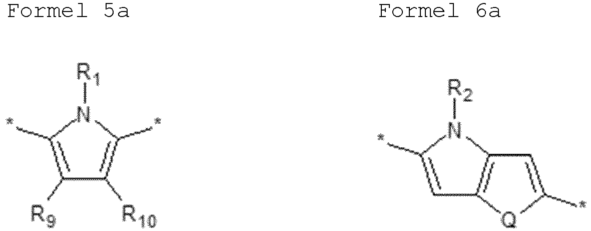 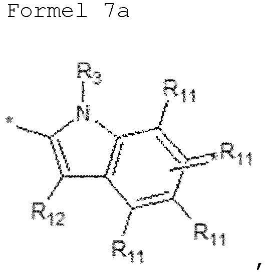
wobei R₉ und R₁₀ jeweils unabhängig voneinander ausgewählt sind aus der Gruppe bestehend aus H, Halogen, bevorzugt F und Cl, CN, Alkyl, Alkenyl, O-Alkyl, S-Alkyl, Aryl, Heteroaryl, bevorzugt sind R₉ und R₁₀ unabhängig voneinander H oder Alkyl,
wobei R₁₁ und R₁₂ jeweils unabhängig voneinander ausgewählt sind aus der Gruppe bestehend aus H, Halogen, bevorzugt F und Cl, CN, Alkyl, Alkenyl, O-Alkyl, S-Alkyl, Aryl, Heteroaryl, bevorzugt sind R₁₁ und R₁₂ H.

Gemäß einer Weiterbildung der Erfindung ist vorgesehen, dass T2 und T3 ausgewählt sind aus der Gruppe bestehend aus: 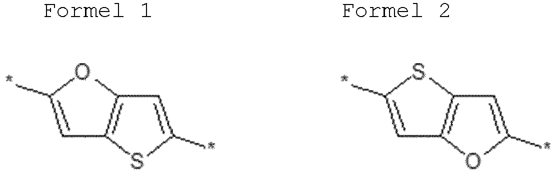 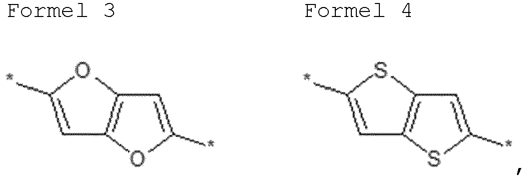 wobei H-Atome der Formeln 1 bis 4 durch Alkyl, O-Alkyl, S-Alkyl, oder Halogen substituiert sein können, bevorzugt sind H-Atome der Formeln 1 bis 4 nicht substituiert, 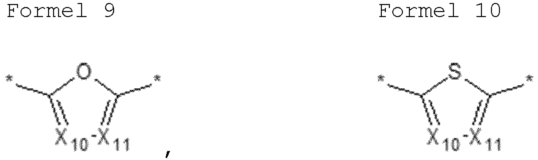 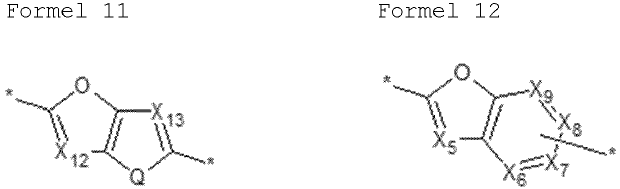 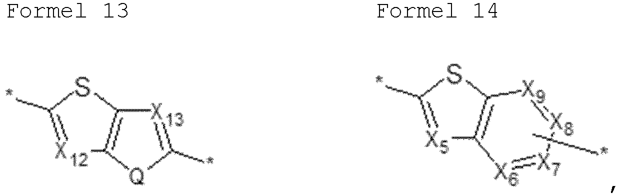 wobei bevorzugt R₆ und R₇ jeweils unabhängig voneinander ausgewählt sind aus der Gruppe bestehend aus H, Alkyl, und Aryl.

In einer bevorzugten Ausführungsform der Erfindung ist T3 ausgewählt aus der Gruppe bestehend aus 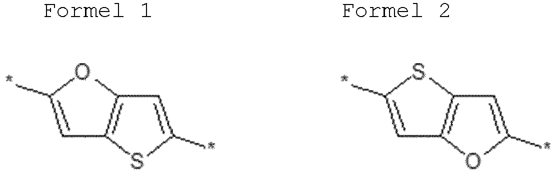 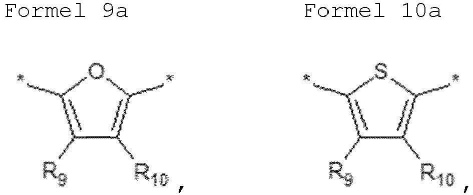 wobei R₉ und R₁₀ jeweils unabhängig voneinander ausgewählt sind aus der Gruppe bestehend aus H, Halogen, bevorzugt F und Cl, CN, Alkyl, Alkenyl, O-Alkyl, S-Alkyl, Aryl, Heteroaryl, bevorzugt sind R₉ und R₁₀ unabhängig voneinander H oder Alkyl.

In einer bevorzugten Ausführungsform der Erfindung sind die Gruppen A1, M, T1, T2, T3 und A2 symmetrisch zueinander ausgebildet.

Gemäß einer Weiterbildung der Erfindung ist vorgesehen, dass A1 ausgewählt ist aus der Gruppe bestehend aus: 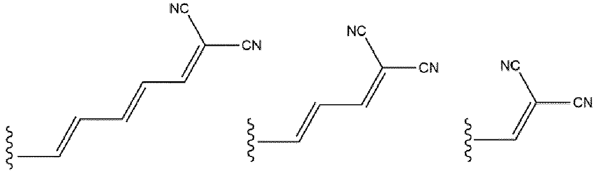 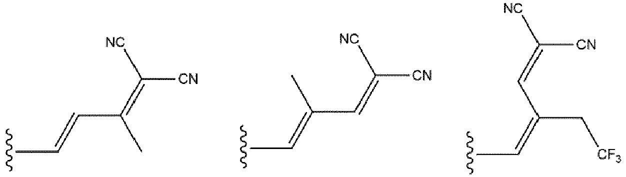 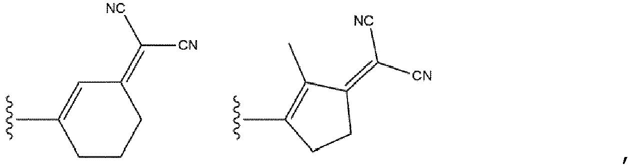 und A2 ausgewählt ist aus der Gruppe bestehend aus:

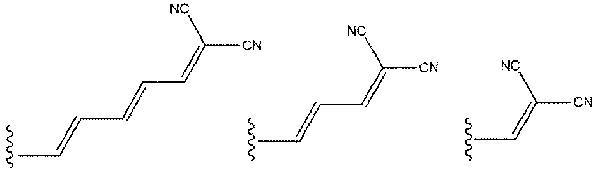

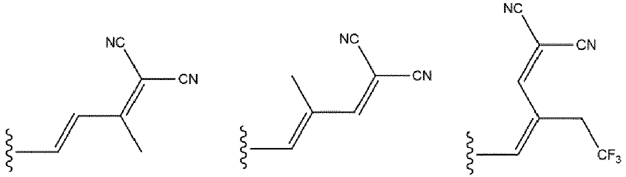

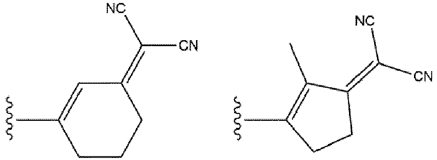

In einer bevorzugten Ausführungsform der Erfindung sind die elektronenziehenden Gruppen A1 und A2 unabhängig voneinander ausgewählt aus: 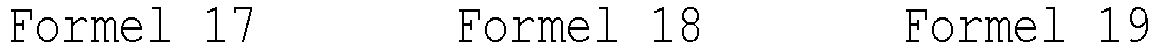 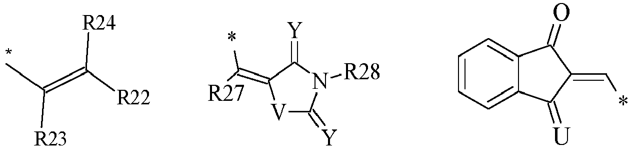
wobei * die Anknüpfung an die Gruppen M, T1, T2 und T3 in der Verbindung der allgemeinen Formel I bezeichnet,
mit R22 und R24 jeweils unabhängig voneinander ausgewählt aus H, CN, und COOR, mit der Maßgabe, dass R22 und R24 nicht beide H sind,
mit R23 jeweils unabhängig voneinander ausgewählt aus der Gruppe bestehend aus H, CN, F, Aryl, Heteroaryl, Alkenyl, Alkinyl, Alkyl, mit V = O oder S; mit Y = O, S oder C(CN)₂; mit U = O, S oder C(CN)₂, mit R27 und R28 jeweils unabhängig voneinander ausgewählt aus der Gruppe bestehend aus H, CN, F, Aryl, Heteroaryl, lkenyl, Alkinyl, Alkyl, wobei für jede der Gruppen A1 und A2 jeweils für jede C=C-Doppelbindung jeweils unabhängig voneinander sowohl das E-Isomer wie auch das Z-Isomer vorliegen kann.

Für jede C=C-Doppelbindung der Formeln 17, 18 und 19 kann also sowohl das E-Isomer ("E" = entgegen; d.h. trans-Konfiguration) wie auch das Z-Isomer ("Z" = zusammen; d.h. cis-Konfiguration) vorliegen, wobei diese Isomere durch eine gedachte Drehung um 180° um die C=C-Doppelbindungsachse gebildet werden.

In einer bevorzugten Ausführungsform der Erfindung ist A1 gleich A2.

Gemäß einer Weiterbildung der Erfindung ist vorgesehen, dass die Parameter a, b, c bevorzugt jeweils unabhängig voneinander 0 oder 1 sind, mit der Maßgabe, dass mindestens einer der Parameter a, b, c gleich 1 ist, bevorzugt sind mindestens zwei der Parameter a, b, c gleich 1; oder der Parameter a gleich 1 oder 2, mit mindestens einem der Parameter b und c gleich 1 oder 2, bevorzugt mit einem der Parameter b oder c gleich 1 oder 2, bevorzugt mit einem der Parameter b oder c gleich 1, insbesondere bevorzugt mit dem Parameter c gleich 1 oder 2.

In einer bevorzugten Ausführungsform der Erfindung sind die Parameter a, b, c jeweils unabhängig voneinander 0, 1 oder 2, mit der Maßgabe, dass mindestens zwei der Parameter a, b, c = 1 sind.

In einer bevorzugten Ausführungsform der Erfindung sind die Parameter a, b, c jeweils unabhängig voneinander 0 oder 1, mit der Maßgabe, dass mindestens zwei der Parameter a, b, c = 1 sind.

In einer besonders bevorzugten Ausführungsform der Erfindung sind a, b und c gleich 1.

In einer bevorzugten Ausführungsform der Erfindung sind die Formeln 1 bis 4 der Gruppe M nicht weiter anelliert.

Gemäß einer Weiterbildung der Erfindung ist vorgesehen, dass die Gruppe M ausgewählt ist aus der Gruppe bestehend aus: 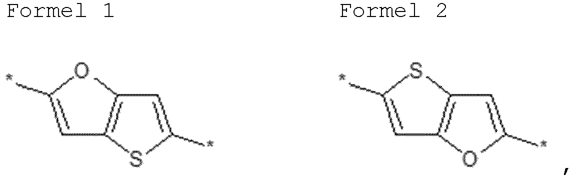 und wobei T1 ausgewählt ist aus der Gruppe bestehend aus 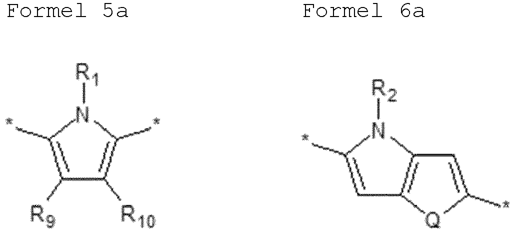 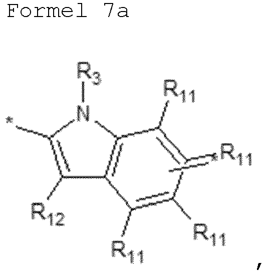
wobei R₉ und R₁₀ jeweils unabhängig voneinander ausgewählt sind aus der Gruppe bestehend aus H, Halogen, bevorzugt F und Cl, CN, Alkyl, Alkenyl, O-Alkyl, S-Alkyl, Aryl, Heteroaryl, bevorzugt sind R₉ und R₁₀ unabhängig voneinander H oder Alkyl,
wobei R₁₁ und R₁₂ jeweils unabhängig voneinander ausgewählt sind aus der Gruppe bestehend aus H, Halogen, bevorzugt F und Cl, CN, Alkyl, Alkenyl, O-Alkyl, S-Alkyl, Aryl, Heteroaryl, bevorzugt sind R₁₁ und R₁₂ H.

Gemäß einer Weiterbildung der Erfindung ist vorgesehen, dass die Verbindung die allgemeinen Formel II aufweist

A1-M-(T1)ₐ-(T3)_{c}-A2 (II),

wobei bevorzugt a und c gleich 1 sind, a gleich 1 und c gleich 2 sind, oder a gleich 2 und c gleich 1 sind.

Gemäß einer Weiterbildung der Erfindung ist vorgesehen, dass die Verbindung ausgewählt ist aus der Gruppe bestehend aus:

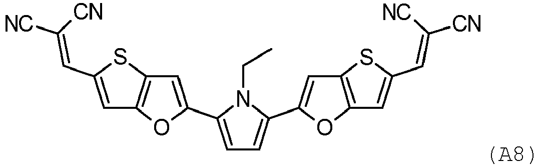

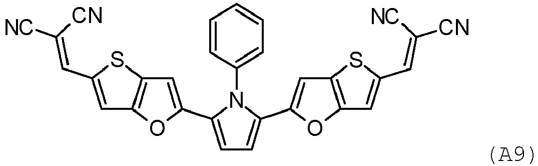

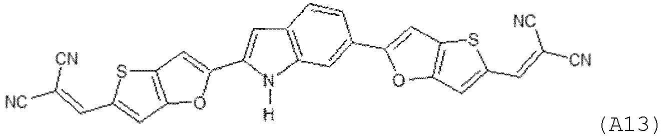

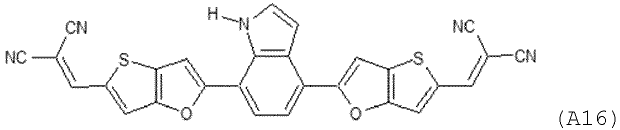

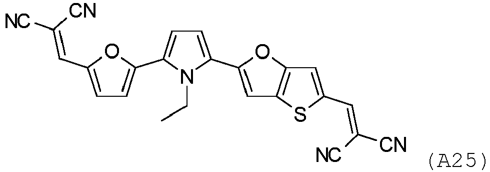

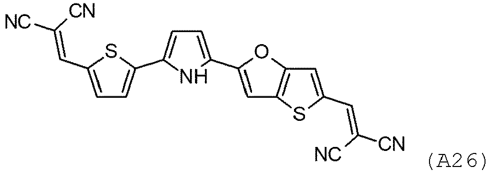

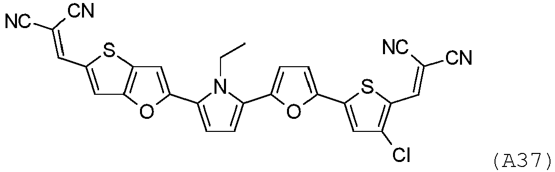

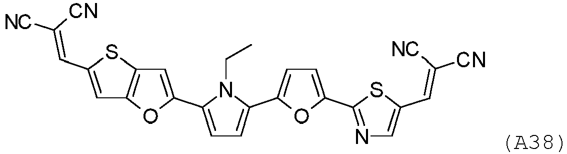

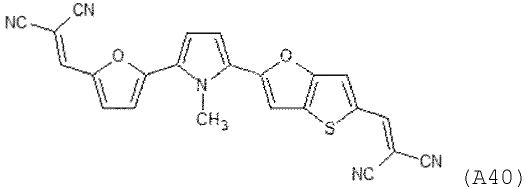

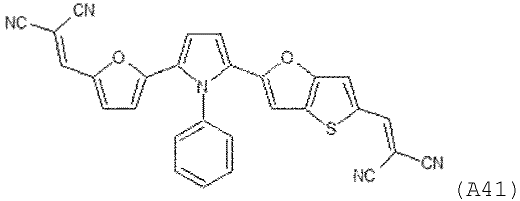

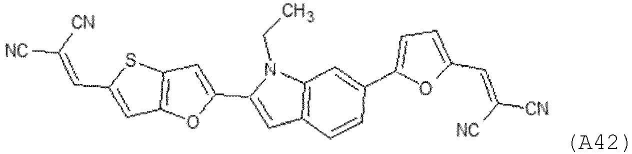

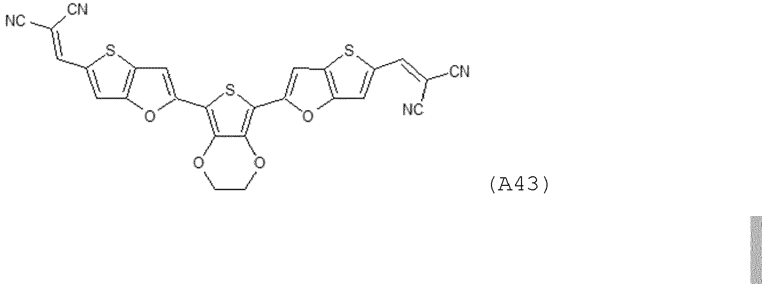

Aufgrund der besonders starken Absorption der erfindungsgemäßen Verbindungen werden besonders gut Exzitonen in Schichten elektronischer Bauelemente gebildet, die diese Verbindungen umfassen, was bei organischen photoaktiven Bauelementen, die diese Verbindungen umfassen, zu höheren Füllfaktoren FF, verbesserter Leerlaufspannung U_{oc} und verbesserter Kurzschlussstromdichte J_{sc} führt. Bei anderen organischen elektronischen Vorrichtungen sind aufgrund der erhöhten Ladungsträgertransporteigenschaften der erfindungsgemäßen Verbindungen ebenfalls bessere elektronische Werte zu erwarten.

Die Aufgabe der vorliegenden Erfindung wird auch gelöst, indem eine Verwendung mindestens einer erfindungsgemäßen Verbindung in einem organischen elektronischen Bauelement, bevorzugt in einem organischen photovoltaischen Element, bereitgestellt wird, insbesondere nach einem der zuvor beschriebenen Ausführungsbeispiele. Dabei ergeben sich für die Verwendung der erfindungsgemäßen Verbindung in einem organischen elektronischen Bauelement insbesondere die Vorteile, die bereits in Zusammenhang mit der Verbindung der allgemeinen Formel (I) erläutert wurden.

In einer bevorzugten Ausführungsform der Erfindung wird die mindestens eine erfindungsgemäße Verbindung in einer Absorberschicht eines photovoltaischen Elements verwendet.

Die Aufgabe der vorliegenden Erfindung wird auch gelöst, indem ein organisches elektronisches Bauelement umfassend eine Elektrode, eine Gegenelektrode und mindestens eine organische photoaktive Schicht zwischen der Elektrode und der Gegenelektrode bereitgestellt wird, wobei die mindestens eine organische photoaktive Schicht mindestens eine erfindungsgemäße Verbindung aufweist, insbesondere nach einem der zuvor beschriebenen Ausführungsbeispiele. Dabei ergeben sich für das organische elektronische Bauelement insbesondere die Vorteile, die bereits in Zusammenhang mit der Verbindung der allgemeinen Formel (I) und der Verwendung der erfindungsgemäßen Verbindung in einem organischen elektronischen Bauelement erläutert wurden.

Unter einem organischen elektronischen Bauelement wird insbesondere ein elektronisches Bauelement verstanden, welches organische leitende oder halbleitende Materialien aufweist, insbesondere Transistoren, organische lichtemittierende Bauelemente, organische photoaktive Vorrichtungen, bei denen in einer photoaktiven Schicht mittels Bestrahlung Exzitonen (Elektron-Loch-Paare) gebildet werden können, bevorzugt Photodetektoren und photovoltaischen Elementen.

Gemäß einer Weiterbildung der Erfindung ist vorgesehen, dass das organische elektronische Bauelement ein organisches photovoltaisches Element, ein OFET, eine OLED oder ein organischer Photodetektor ist. Unter einem photovoltaischen Element wird insbesondere eine Solarzelle verstanden.

In einer bevorzugten Ausführungsform der Erfindung ist das organische elektronische Bauelement als Tandem- oder Mehrfachzelle ausgebildet, wobei mindestens ein weiteres Absorbermaterial, welches in einem anderen spektralen Bereich des Lichtes absorbiert, in einer weiteren Zelle vorhanden ist. Als Tandemzelle wird dabei insbesondere ein elektronisches Bauelement bezeichnet, das aus einem vertikalen Schichtsystem zweier in Serie verschalteter Zellen besteht. Entsprechend wir als eine Mehrfachzelle insbesondere ein elektronisches Bauelement bezeichnet, das aus einem vertikalen Schichtsystem mehrerer in Serie verschalteter Zellen besteht.

In einer bevorzugten Ausführungsform der Erfindung ist die erfindungsgemäße Verbindung der allgemeinen Formel (I) ein Absorbermaterial in einer photoaktiven Schicht eines organischen elektronischen Bauelements. In einer bevorzugten Ausführungsform der Erfindung ist die erfindungsgemäße Verbindung ein Donor in einem Donor-Akzeptor-Heteroübergang, bevorzugt eingesetzt mit einem Akzeptor ausgewählt aus der Gruppe der Fulleren (C60, C70) bzw. Fullerenderivate, Subphthalocyanine, Rylene, Fluorene, Carbazole, Benzothiadiazole, Diketopyrrolopyrrole und Vinazene.

Die photoaktive Schicht kann eine lichtemittierende Schicht sein, die bei Anlegen einer Spannung an die Elektrode und Gegenelektrode durch Rekombination der Löcher (positiven Ladungen) und Elektroden (negative Ladung) Strahlung, z. B. Licht emittiert. Die photoaktive Schicht kann eine lichtabsorbierende Schicht sein, wobei bei Bestrahlung mit einer Strahlung, z. B. sichtbares Licht, UV-Strahlung oder IR-Strahlung, Exzitonen (Elektron-Loch-Paare) gebildet werden. Bei organischen photoaktiven Schichten können insbesondere sogenannte flache Heteroübergänge gebildet werden, bei denen eine flache p-leitende Schicht benachbart zu einer flachen n-leitenden Schicht vorhanden ist und die durch Bestrahlung entweder in der p-leitenden oder n-leitenden Schicht gebildeten Exzitonen an der Grenzfläche zwischen beiden Schichten in Löcher und Elektronen getrennt werden können. Weiterhin kann die photoaktive Schicht auch einen sogenannten Volumenheteroübergang umfassen, bei dem p-leitende und n-leitende Materialien in Form eines interpenetrierenden Netzwerks ineinander übergehen, wobei auch dort die Trennung der durch Bestrahlung gebildeten Exzitonen an den Grenzflächen zwischen p-leitenden und n-leitenden Materialien stattfindet. Exzitonen sind elektrisch neutrale Anregungszustände, die Elektronen-Loch-Paare, die dann in einem weiteren Schritt an einem p-n-Übergang in Elektronen und Löchern getrennt werden. Damit erfolgt die Separation in freie Ladungsträger, die zum elektrischen Stromfluss beitragen. Limitierend ist dabei die Größe der Bandlücke des Halbleiters, entsprechend können nur Photonen absorbiert werden, die eine Energie aufweisen, welche größer als seine Bandlücke ist. Durch das Licht werden immer erst Exzitonen erzeugt, keine freien Ladungsträger, entsprechend ist die rekombinationsarme Diffusion eine für die Höhe des Photostroms wichtige Komponente. Die Exzitonendiffusionslänge muss dabei die typische Eindringtiefe des Lichtes überschreiten, damit ein möglichst großer Teil des Lichtes elektrisch nutzbar ist. Die Exzitonen gelangen durch Diffusion an eine Grenzfläche, wo Elektronen und Löcher voneinander getrennt werden. Das Material, welches die Elektronen aufnimmt, wird als Akzeptor und das Material, welches die Löcher aufnimmt, wird als Donor oder Donator bezeichnet.

Ein bereits literaturbekannter Aufbau eines gängigen organischen photovoltaischen Elements besteht in einer pin- oder nip-Dioden [Martin Pfeiffer, "Controlled doping of organic vacuum deposited dye layers: basics and applications", PhD thesis TU-Dresden, 1999 und WO2011/161108A1]: eine pin Solarzelle besteht dabei aus einem Träger/Substrat mit daran anschließendem meist transparentem Grundkontakt, p-Schicht(en), i-Schicht(en), n-Schicht(en) und einem Deckkontakt. Eine nip-Zelle besteht aus einem Träger/Substrat mit daran anschließendem meist transparentem Grundkontakt, n-Schicht(en), i-Schicht(en), p-Schicht(en) und einem Deckkontakt. Dabei bedeutet n bzw. p-Dotierung, die zu einer Erhöhung der Dichte freier Elektronen bzw. Löcher im thermischen Gleichgewichtszustand führt. Damit sind solche Schichten primär als Transportschichten zu verstehen. Die Bezeichnung i-Schicht kennzeichnet eine undotierte oder intrinsiche Schicht. Eine oder mehrere i-Schichten können dabei aus einem Material (planar Heterojunctions) als auch aus einer Mischung zweier oder mehrerer Materialien bestehen (bulk heterojunctions), die ein interpenetrierendes Netzwerk aufweisen.

WO2011161108A1 offenbart ein photoaktives Bauelement mit einer Elektrode und einer Gegenelektrode, wobei zwischen den Elektroden zumindest ein organisches Schichtsystem angeordnet ist, weiterhin mit mindestens zwei photoaktiven Schichtsystemen und zwischen den photoaktiven Schichtsystemen zumindest zwei verschiedene Transportschichtsysteme des gleichen Ladungsträgertyps. Die Herstellung derartiger organischer elektronischer Bauelemente, insbesondere organischer photovoltaischer Elemente, ist dem Fachmann hinreichend bekannt, insbesondere aus der genannten Literatur.

Die Erfindung wird im Folgenden anhand der Zeichnungen näher erläutert. Dabei zeigen:
Fig. 1 eine schematische Darstellung eines Ausführungsbeispiels eines organischen elektronischen Bauelements im Querschnitt;
Fig. 2 eine Strom-Spannungskurve eines organischen elektronischen Bauelementes mit der Verbindung A8;
Fig. 3 eine Strom-Spannungskurve eines organischen elektronischen Bauelementes mit der Verbindung A25; und m
Fig. 4 in einer Übersicht die Parameter Füllfaktor FF, Leerlaufspannung Uoc, und Kurzschlussstrom Jsc organischer elektronischer Bauelemente mit erfindungsgemäßen Verbindungen und nicht-erfindungsgemäßen Verbindungen im direkten Vergleich.

### Ausführungsbeispiele

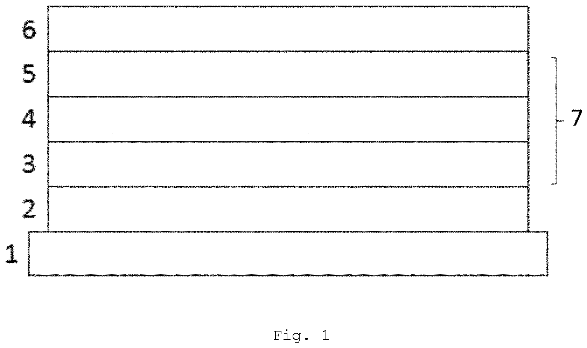

Fig. 1 zeigt eine schematische Darstellung eines Ausführungsbeispiels eines organischen elektronischen Bauelements im Querschnitt.

Das erfindungsgemäße organische elektronische Bauelement weist ein Schichtsystem 7 auf, wobei zumindest eine Schicht des Schichtsystems 7 eine erfindungsgemäße Verbindung der allgemeinen Formel I aufweist.

Das organische elektronische Bauelement umfasst eine erste Elektrode 2, eine zweite Elektrode 6 und ein Schichtsystem 7, wobei das Schichtsystem 7 zwischen der ersten Elektrode 2 und der zweiten Elektrode 6 angeordnet ist. Dabei weist mindestens eine Schicht des Schichtsystems 7 mindestens eine erfindungsgemäße Verbindung der allgemeinen Formel I auf.

Das Schichtsystem 7 weist eine photoaktiven Schicht 4 auf, bevorzugt eine lichtabsorbierende photoaktive Schicht 4, wobei die photoaktive Schicht 4 die mindestens eine erfindungsgemäße Verbindung aufweist.

In einem Ausführungsbeispiel weist das organische photovoltaische Element ein Substrat 1 auf, z.B. aus Glas, auf dem sich eine Elektrode 2 befindet, die z.B. ITO umfasst. Darauf angeordnet ist das Schichtsystem 7 mit einer elektronentransportierenden Schicht 3 (ETL) sowie einer photoaktiven Schicht 4 mit mindestens einer erfindungsgemäßen Verbindung, einem p-leitenden Donor-Material, und einem n-leitenden Akzeptor-Material, z. B. C60 Fulleren, entweder als flacher Heteroübergang (planar heterojunction) oder als Volumenheteroübergang (bulk heterojunction). Darüber angeordnet befindet sich eine p-dotierte Lochtransportschicht 5 (HTL), und eine Elektrode 6 aus Gold oder Aluminium.

Die Herstellung des Schichtsystems, insbesondere einzelner Schichten, des Bauelements kann durch Verdampfen der Verbindungen im Vakuum, mit oder ohne Trägergas oder prozessieren einer Lösung oder Suspension, wie zum Beispiel beim Coaten oder Drucken geschehen. Einzelne Schichten können ebenso durch Sputtern aufgetragen werden. Vorteilhaft ist die Herstellung der Schichten durch Verdampfen im Vakuum, wobei das Trägersubstrat erwärmt sein kann. Die organischen Materialien werden dabei in Form dünner Filme oder kleiner Volumen auf die Folien aufgedruckt, aufgeklebt, gecoated, aufgedampft oder anderweitig angebracht. Für die Herstellung der dünnen Schichten kommen ebenso alle Verfahren in Betracht, die auch für Elektronik auf Glas, keramischen oder halbleitenden Trägern verwendet werden.

Die chemische Verbindung der allgemeinen Formel I weist folgende Struktur auf:

A1-M-(T1)ₐ-(T2)_{b}-(T3)_{c}-A2 (I)

mit den Parametern a, b, c jeweils unabhängig voneinander 0, 1 oder 2, mit der Maßgabe, dass mindestens einer der Parameter a, b, c = 1 oder 2 ist,
mit der Gruppe M ausgewählt aus der Gruppe bestehend aus: 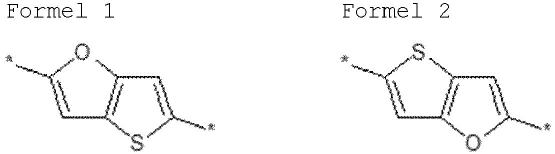 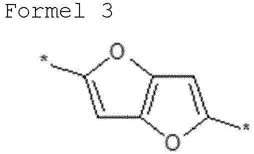
wobei * die Anknüpfung an die Gruppen T1, T2, T3, A1 und A2 bezeichnet, wobei H-Atome der Formeln 1 bis 4 durch Alkyl, O-Alkyl, S-Alkyl, oder Halogen substituiert sein können;
mit den Gruppen T1, T2 und T3 unabhängig voneinander ausgewählt aus der Gruppe bestehend aus: 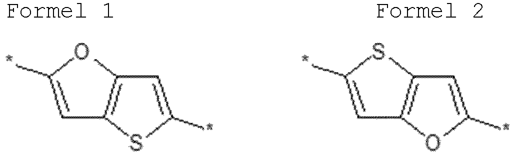 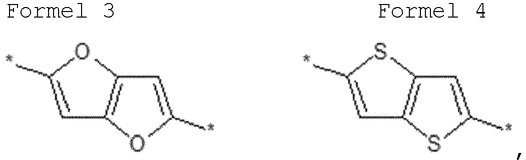
wobei H-Atome der Formeln 1 bis 4 durch Alkyl, O-Alkyl, S-Alkyl, oder Halogen substituiert sein können, 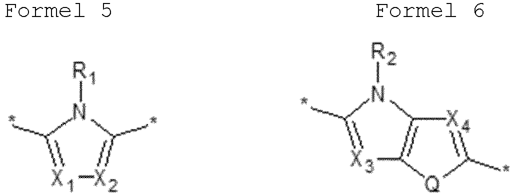 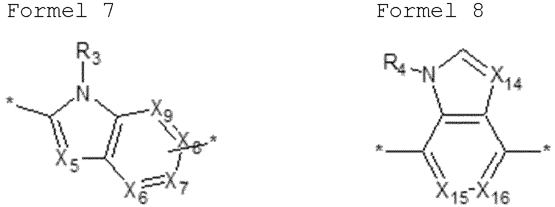
mit R1 bis R4 jeweils unabhängig voneinander ausgewählt aus der Gruppe bestehend aus H, Alkyl, Alkenyl, Aryl und Heteroaryl; 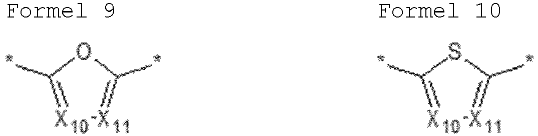 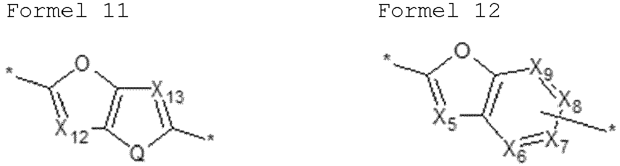 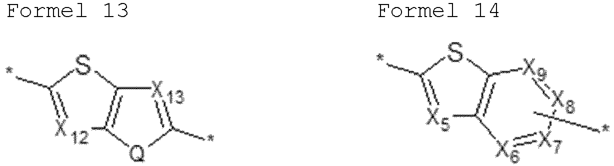 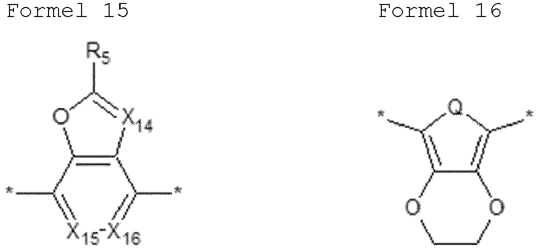 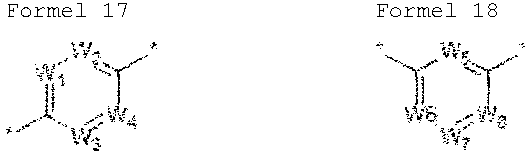
mit X₁ bis X₁₆ unabhängig voneinander ausgewählt aus N oder CR₆, mit der Maßgabe, dass in den Formeln 7, 12 und 14 jeweils eine Gruppe aus X₆ bis X₉ die Anknüpfung * an eine weitere Gruppe in der Verbindung der allgemeinen Formel I bezeichnet,
mit Q ausgewählt aus der Gruppe bestehend aus O, S, und NR₇,
mit W₁ bis W₈ jeweils unabhängig voneinander ausgewählt aus N und CR₈, wobei R₆, R₇ und R₈ jeweils unabhängig voneinander ausgewählt sind aus der Gruppe bestehend aus H, CN, Alkyl, Alkenyl, O-Alkyl, S-Alkyl, Aryl, und Heteroaryl;
wobei die elektronenziehenden Gruppen A1 und A2 unabhängig voneinander elektronenziehende Gruppen mit zumindest einer C=C-Doppelbindung sind, und wobei * die Anknüpfung an eine weitere Gruppe M, T1, T2, T3, und A2 in der Verbindung der allgemeinen Formel I bezeichnet.

In einer Ausgestaltung der Erfindung ist T1 unabhängig voneinander ausgewählt aus der Gruppe bestehend aus: 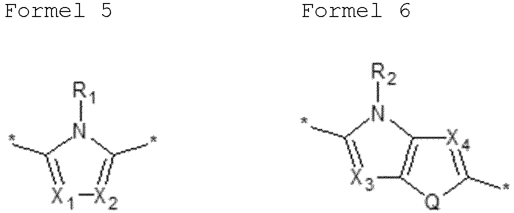 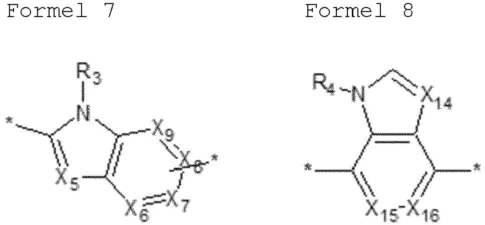 wobei bevorzugt X₁ und X₂ und/oder X₃ und X₄ CR₆ sind, bevorzugt ist R₆ ausgewählt aus H und Alkyl; und/oder wobei bevorzugt X₅ CR₆ ist mit R₆ H oder Alkyl, und/oder wobei bevorzugt X₆ bis X₉ CR₆ sind; und/oder wobei bevorzugt R₁ bis R₃ unabhängig voneinander H, Alkyl oder Aryl sind; und/oder wobei bevorzugt R₄ H oder Alkyl ist.

In einer weiteren Ausgestaltung der Erfindung sind T2 und T3 ausgewählt aus der Gruppe bestehend aus: 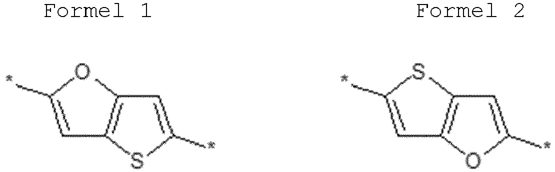 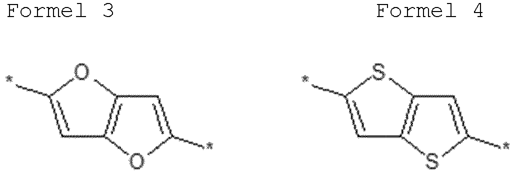 wobei H-Atome der Formeln 1 bis 4 durch Alkyl, O-Alkyl, S-Alkyl, oder Halogen substituiert sein können, bevorzugt sind H-Atome der Formeln 1 bis 4 nicht substituiert, 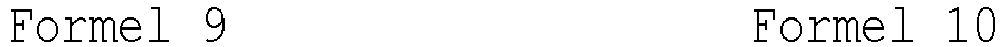 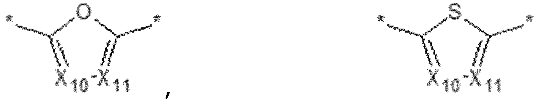 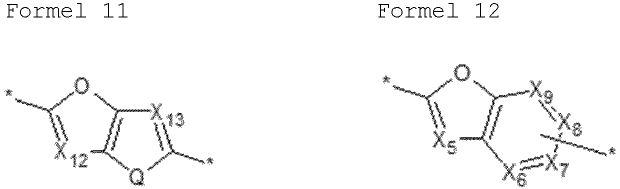 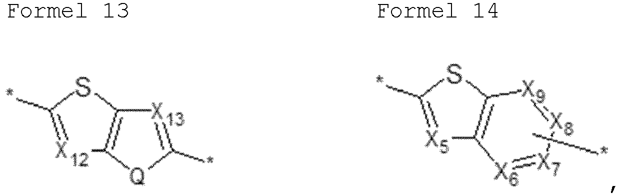 wobei bevorzugt R₆ und R₇ jeweils unabhängig voneinander ausgewählt sind aus der Gruppe bestehend aus H, Alkyl, und Aryl.

In einer weiteren Ausgestaltung der Erfindung ist A1 ausgewählt aus der Gruppe bestehend aus: 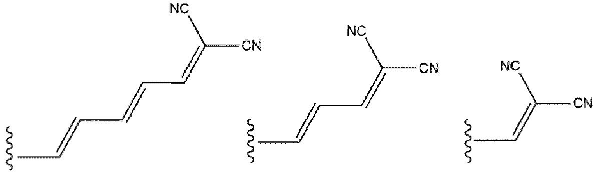 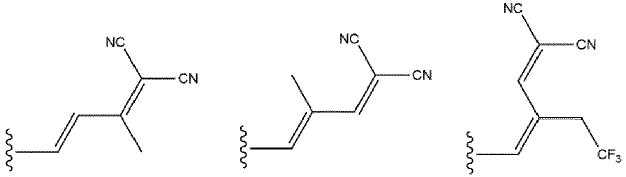 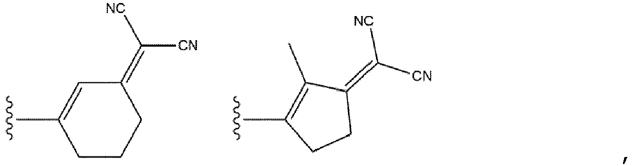 und ist A2 ausgewählt aus der Gruppe bestehend aus:

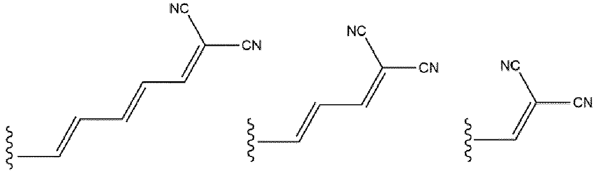

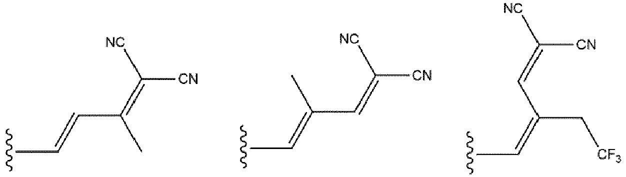

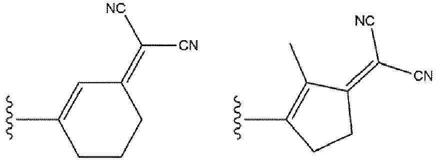

In einer weiteren Ausgestaltung der Erfindung sind die Parameter a, b, c bevorzugt jeweils unabhängig voneinander 0 oder 1, mit der Maßgabe, dass mindestens einer der Parameter a, b, c gleich 1 ist, bevorzugt sind mindestens zwei der Parameter a, b, c gleich 1; oder ist der Parameter a gleich 1 oder 2, mit mindestens einem der Parameter b und c gleich 1 oder 2, bevorzugt mit einem der Parameter b oder c gleich 1 oder 2, bevorzugt mit einem der Parameter b oder c gleich 1, insbesondere bevorzugt mit dem Parameter c gleich 1 oder 2.

In einer weiteren Ausgestaltung der Erfindung ist die Gruppe M ausgewählt aus der Gruppe bestehend aus: 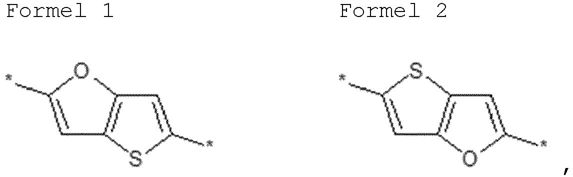 und wobei T1 ausgewählt ist aus der Gruppe bestehend aus 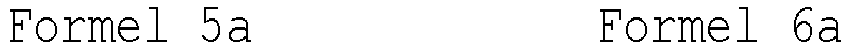 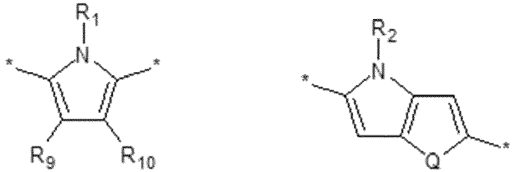 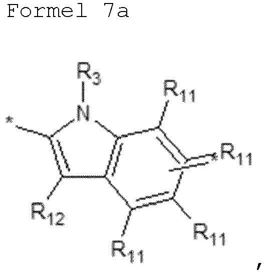
wobei R₉ und R₁₀ jeweils unabhängig voneinander ausgewählt sind aus der Gruppe bestehend aus H, Halogen, bevorzugt F und Cl, CN, Alkyl, Alkenyl, O-Alkyl, S-Alkyl, Aryl, Heteroaryl, bevorzugt sind R₉ und R₁₀ unabhängig voneinander H oder Alkyl,
wobei R₁₁ und R₁₂ jeweils unabhängig voneinander ausgewählt sind aus der Gruppe bestehend aus H, Halogen, bevorzugt F und Cl, CN, Alkyl, Alkenyl, O-Alkyl, S-Alkyl, Aryl, Heteroaryl, bevorzugt sind R₁₁ und R₁₂ H.

In einer weiteren Ausgestaltung der Erfindung weist die Verbindung die allgemeinen Formel II auf

A1-M-(T1)ₐ-(T3)_{c}-A2 (II),

wobei bevorzugt a und c gleich 1 sind, a gleich 1 und c gleich 2 sind, oder a gleich 2 und c gleich 1 sind.

In Tabelle 1 ist eine Übersicht über die thermischen Eigenschaften erfindungsgemäßer Verbindungen dargestellt (Schmelzpunkte und thermisches Budget). Die thermischen Eigenschaften zeigen, dass erfindungsgemäße Verbindungen zur Herstellung elektronischer Bauelemente mittels Prozessierung im Vakuum geeignet sind.

**Tabelle 1**

| **Verbindung** | **Smp./°C^{a}** | **Thermisches Budget/°C** ^{b} |
|---|---|---|
| A8 | 339 | 74 |
| A9 | 373 | 118 |
| A16 | 432 | 122 |
| A25 | 282 | 62 |
| A26 | 367 | 107 |
| A37 | 296 | 46 |
| A38 | 267 | 27 |

| | | |
|---|---|---|
| ^{a} onset DSC ^{b} Differenz zwischen Schmelpunkt und Verdampfungstemperatur | | |

Tabelle 2 zeigt in einer Übersicht Absorptionsmaxima (in nm und eV im Lösungsmittel (LM)) erfindungsgemäßer Verbindungen.

**Tabelle 2**

| **Verbindung** | **Struktur** | **Smp./°C^{a}** | **λmax (LM) /nm^{b}** | **in eV** |
|---|---|---|---|---|
| A8 | 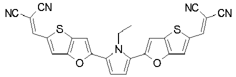 | 339 | 529 | 2,34 |
| A9 | 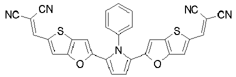 | 373 | 574 | 2,16 |
| A13 | 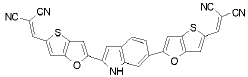 | | 540 | 2,30 |
| A16 | 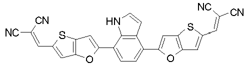 | 432 | 535 | 2,32 |
| A25 | 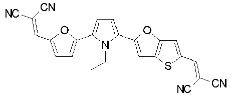 | 282 | 543 | 2,28 |
| A26 | 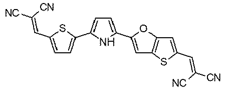 | 367 | 543 | 2,28 |
| A37 | 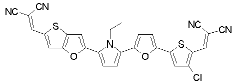 | 296 | 571 | 2,17 |
| A38 | 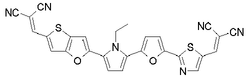 | 267 | 543 | 2,28 |

| | | | | |
|---|---|---|---|---|
| ^{a} onset DSC (dynamische Differenzkalorimetrie; Beginn des Schmelzbereiches; Extrapolierte Anfangstemperatur (Schnittpunkt Wendetangente-Basislinie) ^{b} in Dichlormethan wenn nicht anders vermerkt | | | | |

Überraschenderweise wurde gefunden, dass die erfindungsgemäßen Verbindungen eine besonders starke Absorption aufweisen, d.h. eine hohe optische Dichte am Absorptionsmaximum und/oder ein hohes Integral über die optische Dichte im sichtbaren Spektralbereich im Vergleich zu ähnlichen Verbindungen außerhalb des hier beanspruchten Bereichs.

Es konnte darüber hinaus gezeigt werden, dass die erfindungsgemäßen Verbindungen nicht nur Licht absorbieren sondern auch rückstandsfrei im Vakuum verdampft werden können. Durch sehr gute Ladungstransporteigenschaften und gute Absorptionseigenschaften können hohe Photoströme mit erzeugt werden. Damit können sehr gut kombinierte Tandem-/ Tripel-/ Quadrupel-/ oder Multijunction-Zellen hergestellt werden.

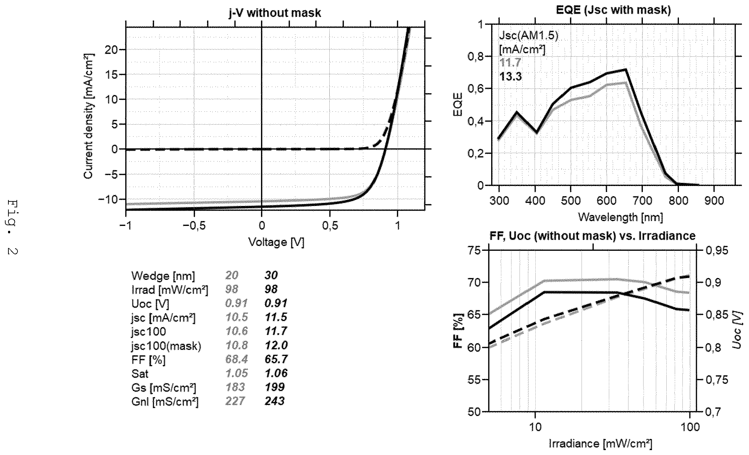

Fig. 2 zeigt eine Strom-Spannungskurve eines organischen elektronischen Bauelementes mit der Verbindung A8. Das organische elektronische Bauelement ist in diesem Ausführungsbeispiel ein organisches photovoltaisches Element.

Die photovoltaischen Parameter Uoc, Jsc und FF beziehen sich jeweils auf photovoltaische Elemente mit 30 nm dicker Mischschicht aus dem jeweiligen Donormaterial dieser Verbindungen und Fulleren C60 als photoaktiver Schicht als bulk-Heterojunction (BHJ) ausgebildet auf Glas mit dem Aufbau:
ITO/ C60 (15 nm)/ Verbindung A8:C60 (30nm, 3:2, 40°C)/ NHT169 (10nm)/ NHT169:NDP9 (30nm, 9,1%wt)/ NDP9 (1nm)/ Au (50nm), wobei die photoaktive Schicht ein Volumenheteroübergang (bulk heterojunction BHJ) ist. Dabei ist ITO Indiumzinnoxid, NDP9 ein kommerzieller p-Dotand der Novaled GmbH, und NHT169 ein kommerzieller Lochleiter der Novaled GmbH. Die Parameter der Zelle wurden gemessen unter AM1.5 Beleuchtung (Am = Air Mass; bei diesem Spektrum beträgt die globale Strahlungsleistung 1000 W/m²; AM = 1,5 als Standardwert für die Vermessung von Solarmodulen).

In dem organischen photovoltaischen Element mit der Verbindung A8 beträgt der Füllfaktor FF 65,7 %, die Leerlaufspannung Uoc 0,91 V und der Kurzschlussstrom Jsc 13,3 mA/cm2. Der Zellwirkungsgrad des photovoltaischen Elements mit Verbindung A8 beträgt 8,0%.

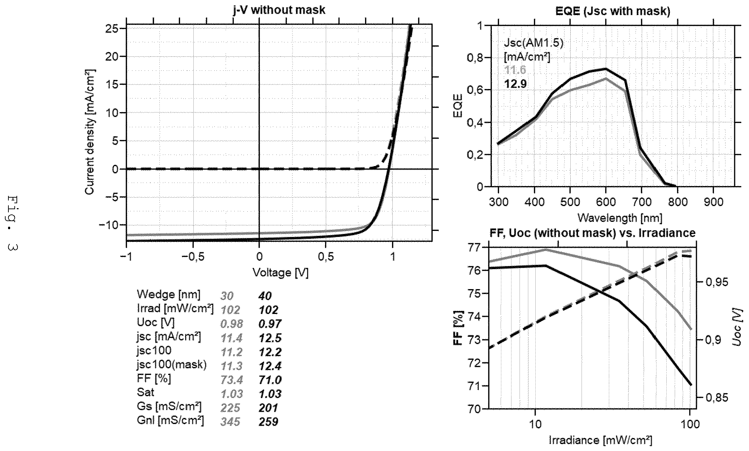

Fig. 3 zeigt eine Strom-Spannungskurve eines organischen elektronischen Bauelementes mit der Verbindung A25. Das organische elektronische Bauelement ist in diesem Ausführungsbeispiel ein organisches photovoltaisches Element.

Der Aufbau des photovoltaischen Elements entspricht dem aus Fig. 2:
ITO/ C60 (15 nm)/ Verbindung A25:C60 (30nm, 3:2, 40°C)/ NHT049 (10nm)/ NHT049:NDP9 (30nm, 9,1%wt)/ NDP9 (1nm)/ Au (50nm), wobei die photoaktive Schicht ein Volumenheteroübergang (bulk heterojunction BHJ) ist. Dabei ist ITO Indiumzinnoxid, NDP9 ein kommerzieller p-Dotand der Novaled GmbH, und NHT049 ein kommerzieller Lochleiter der Novaled GmbH. Die Parameter der Zelle wurden gemessen unter AM1.5 Beleuchtung (Am = Air Mass; bei diesem Spektrum beträgt die globale Strahlungsleistung 1000 W/m²; AM = 1,5 als Standardwert für die Vermessung von Solarmodulen).

In dem photovoltaischen Element mit der Verbindung A25 beträgt der Füllfaktor FF 73,4 %, die Leerlaufspannung Uoc 0,98 V und der Kurzschlussstrom Jsc 11,6 mA/cm2. Der Zellwirkungsgrad des photovoltaischen Elements mit Verbindung A25 beträgt 8,3%.

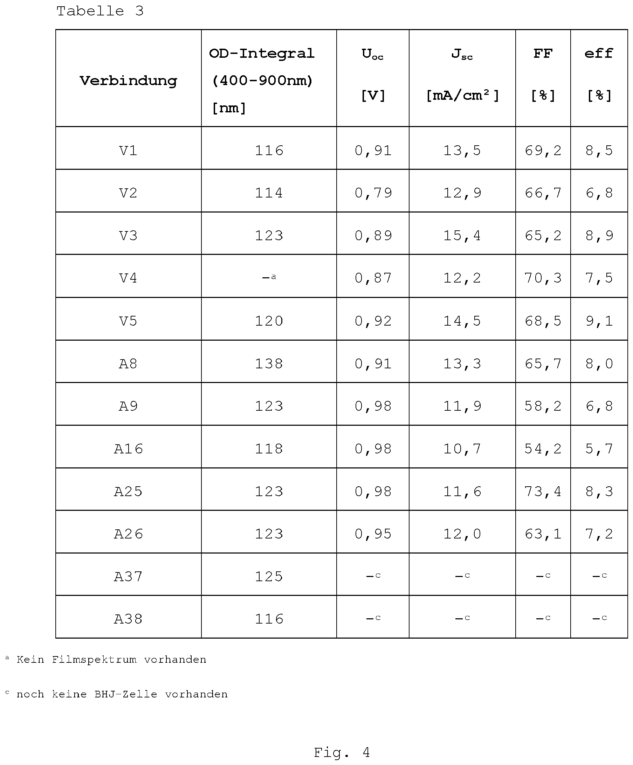

Fig. 4 zeigt in einer Übersicht die Parameter Füllfaktor FF, Leerlaufspannung Voc, und Kurzschlussstrom Jsc organischer elektronischer Bauelemente mit erfindungsgemäßen Verbindungen und nicht-erfindungsgemäßen Verbindungen im direkten Vergleich. Die organischen elektronischen Bauelemente sind in diesem Ausführungsbeispiel organische photovoltaische Elemente.

Der Aufbau der photovoltaischen Elemente entspricht dem Aufbau des photovoltaischen Elements aus Fig. 2 und aus Fig. 3. Fig.4 zeigt Parameter erfindungsgemäßer Verbindungen und nicht-erfindungsgemäßer Verbindungen im direkten Vergleich. Die photovoltaischen Parameter beziehen sich jeweils auf photovoltaische Elemente mit 30 nm dicker Mischschicht aus dem jeweiligen Donormaterial der entsprechenden Verbindung und Fulleren C60 auf Glas mit dem Aufbau:
ITO/ C60 (15 nm)/ Verbindung:C60 (30nm, 3:2, 40°C)/ NHT049 oder NHT169 (10nm)/ NHT049 oder NHT169:NDP9 (30nm, 9,1%wt)/ NDP9 (1nm)/ Au (50nm), wobei die photoaktive Schicht ein Volumenheteroübergang (bulk heterojunction BHJ) ist.

Die nicht-erfindungsgemäßen Vergleichsverbindungen V1 bis V5 sind folgende:

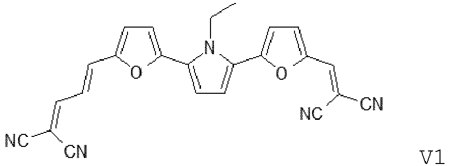

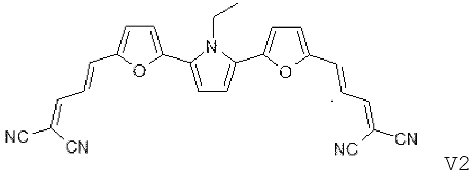

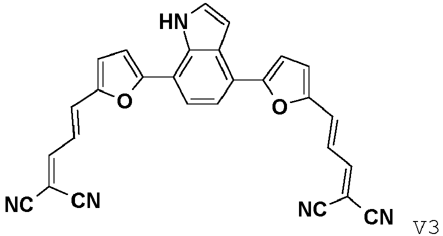

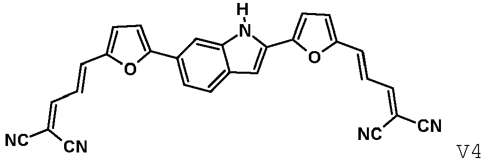

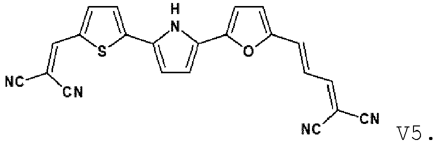

Die Parameter organischer elektronischer Bauelemente Parameter mit erfindungsgemäßen Verbindungen und nicht-erfindungsgemäßen Verbindungen sind in Tabelle 3 (Fig. 4) zusammengefasst. Tabelle 3 zeigt das optische Integral im sichtbaren Bereich (OD-Integral), sowie U_{oc}, J_{sc}, FF und die Effizienz. Die erfindungsgemäßen Verbindungen zeigen im Vergleich zu den nicht-erfindungsgemäßen Verbindungen (V1 bis V5) insbesondere eine erhöhte Leerlaufspannung Uoc von 0,91 V und größer, insbesondere in einem Spektralbereich von 400 nm bis 600 nm.

Im Folgenden sind exemplarisch Synthesen konkreter Ausführungsbeispiele erfindungsgemäßer Verbindungen dargestellt.

Die Synthese der allgemeinen Verbindung (I) kann nach einer der im Folgenden beschriebenen Methoden erfolgen. Diese soll hier als beispielhafte Darstellung dienen und kann in der Reihenfolge ihrer einzelnen Schritte variiert, oder durch andere bekannte Methoden abgewandelt werden. Auch die Zusammenfassung einzelner Reaktionsschritte oder die Veränderung von Teilen der Syntheseroute ist möglich.

### Synthese Gruppe 1:

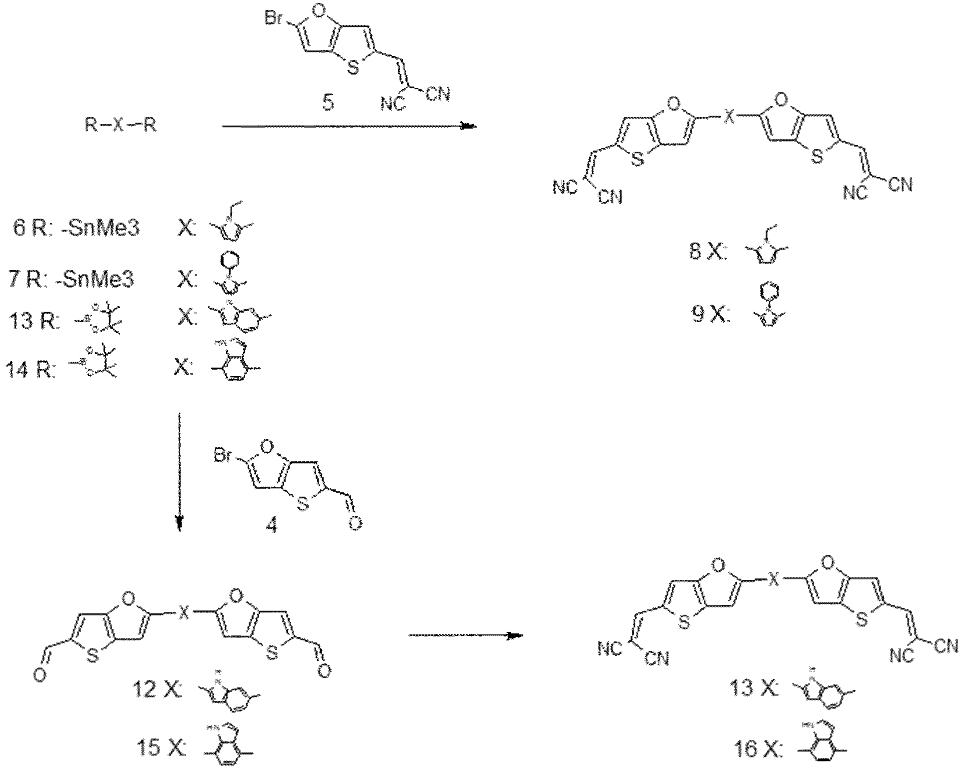

### Synthese von Verbindung A8 und Verbindung A9

### Synthese of 2,2'-{(1-ethyl-1H-pyrrole-2,5-diyl)bis[(thieno[3,2-b]furan-2,5-diyl)methanylylidene]}dipropanedinitrile (A8) und 2,2'-{(1-phenyl-1H-pyrrole-2,5-diyl)bis[(thieno[3,2-b]furan-2,5-diyl) methanylylidene]}dipropanedinitrile (A9)

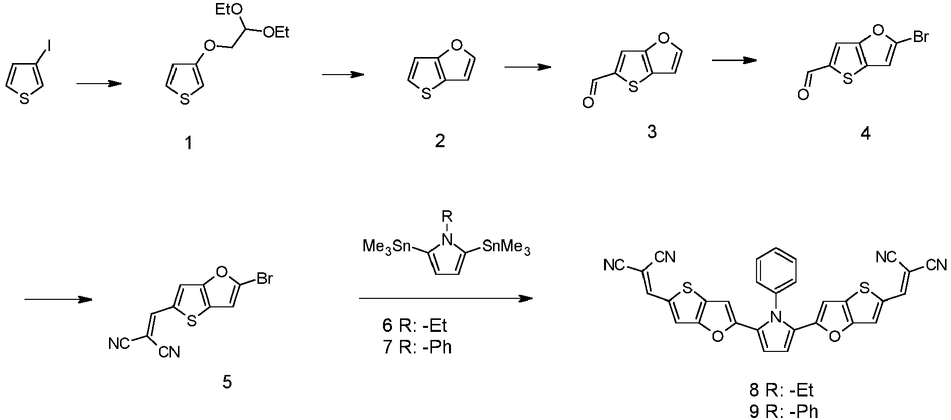

Verbindungen 6 und 7 könnten synthetisiert werden wie beschrieben bei Fitzner, Roland; Gerdes, Olga; Hildebrandt, Dirk; D'Souza, Daniel; Mattersteig, Gunter; Weiss, Andre From Eur. Pat. Appl. (2017), EP3188270A1.

### Synthese von 3-(2,2-diethoxyethoxy)thiophene (1)

3,28 g (15 mmol) von 3-Iodthiophen, 4,11 g (30 mmol) von 2,2-Diethoxyethanol und 601 mg (3 mmol) von 1,10-Phenanthrolin Monohydrat wurden in 7,5 ml Toluol gelöst. 286 mg (1,5 mmol) von Kupfer(I)-iodid und 9,77 g (30 mmol) von Cäsiumcarbonat wurden dazu gegeben. Das Reaktionsgemisch wurde für 64 h zum Sieden erhitzt. Nachdem das Gemisch abgekühlt war, wurde es mit Diethylether versetzt und durch Silikagel filtriert. Das Filtrat wurde eingeengt und das Rohprodukt wurde chromatographiert am Silikagel in Diethylether:Petrolether 1:9 (Rf 0,43). Dabei wurde 2,31 g (71% Ausbeute) von 3-(2,2-Diethoxyethoxy)thiophene 1 isoliert. GC-MS(EI) m/z: 216 (20%), 171 (20%), 125 (60%), 103 (100%). 1H-NMR in CDCl3, ppm: 7,16 (dd, 1H), 6,78 (dd, 1H), 6,26 (dd, 1H), 4,82 (t, 1H), 3,99 (d, 2H), 3,75 (m, 2H), 3,62 (m, 2H), 1,25 (t, 6H).

### Synthese von thieno[3,2-b]furan (2)

2,31 g (10,7 mmol) von 3-(2,2-diethoxyethoxy)thiophene 1 wurden in 54 ml THF gelöst und 2,4 g von Amberlyst 15 wurden zugegeben. Das Gemisch wurde für 10 h zum Sieden erhitzt. Nachdem das Gemisch abgekühlt war, wurde es abdekantiert. Im Rückstand gebliebene Amberlyst^{®} 15 wurde in heißem THF ausgerührt und anschließend wieder abdekantiert. Die vereinten organischen Phasen wurden mit Petrolether verdünnt, mit ges. NaCl-Lösung gewaschen und über Natriumsulfat getrocknet. Nachdem die Lösungsmittel am Rotationsverdampfer entfernt worden sind, wurde das Rückstand per Säulenchromatographie am Silikagel in Petrolether gereinigt. Dabei wurde 448 mg (34 % Ausbeute) von Thieno[3,2-b]furan 2 isoliert. GC-MS(EI) m/z: 124 (100%). 1H-NMR in d6-Aceton, ppm: 7,74 (dd, 1H), 7,41 (dd, 1H), 7,15 (dd, 1H), 6,90 (dd, 1H) .

### Synthese von thieno[3,2-b]furan-5-carbaldehyde (3)

10,1 g (81,1 mmol) von thieno[3,2-b]furan 2 wurden in 143 ml trock. THF gelöst und die Lösung auf -78°C abgekühlt. 32,4 ml (81,1 mmol) von 2,5-M-nBuLi wurden langsam dazu gegeben und das Gemisch wurde für 2 h bei -78°C gerührt. 9,27 g (81,1 mmol) von N-Formylpiperidin wurde zum Reaktionsgemisch zugegeben und das Reaktionsgemisch wurde über Nacht auf Raumtemperatur erwärmt. Das Reaktionsgemisch wurde mit ges. NaHCO3 Lösung versetzt und mit Dichlormethan extrahiert. Organische Phase wurde mit Wasser und ges. NaCl-Lösung gewaschen, und über Natriumsulfat getrocknet. Rohprodukt wurde mittels Säulenchromatographie am Silikagel in Petrolether:Dichlormethan 1:3 gereinigt. Dabei wurde 7,8 g (63 % Ausbeute) von Thieno[3,2-b]furan-5-carbaldehyde 3 isoliert. GC-MS(EI) m/z: 152 (100 %), 123 (20 %). 1H-NMR in d6-Aceton, ppm: 9,95 (s, 1H), 8,06 (dd, 1H), 8,04 (dd, 1H), 7,09 (dd, 1H).

### Synthese von 2-bromothieno[3,2-b]furan-5-carbaldehyde (4)

1,52 g (10 mmol) von 2-bromothieno[3,2-b]furan-5-carbaldehyde 4 wurden in 23 ml trock. DMF gelöst. 2,72 g (15 mmol) von N-Bromsuccinimid wurden in kleinen Portionen zugegeben und das Reaktionsgemisch wurde 1 h bei Raumtemperatur gerührt. Anschließend, wurde das Gemisch auf Eis gegeben und ausgefallener Niederschlag abfiltriert. Rohprodukt wurde aus Ethanol umkristallisiert. Dabei wurde 1,74 g (75% Ausbeute) von 2-bromothieno[3,2-b]furan-5-carbaldehyde 4 isoliert. GC-MS(EI) m/z: 231 (100%). 1H-NMR in d6-Aceton, ppm: 9,97 (s, 1H), 8,04 (s, 1H), 7,19 (s, 1H).

### Synthese von [(2-bromothieno[3,2-b]furan-5-yl)methylidene]propanedinitrile (5)

690 mg (2,98 mmol) von [(2-bromothieno[3,2-b]furan-5-yl)methylidene]propanedinitrile 5 und 394 mg (5,97 mmol) von Malodinitril wurden in 8 ml Ethanol gelöst und 13,6 mg (0,15 mmol) β-Alanin dazu gegeben. Das Reaktionsgemisch wurde 2 h zum Sieden erhitzt. Nachdem das Gemisch auf Raumtemperatur abgekühlt war, wurde Niederschlag abfiltriert und mit wenig Ethanol gewaschen. Dabei wurde 555 mg (67% Ausbeute) von [(2-bromothieno[3,2-b]furan-5-yl)methylidene]propanedinitrile 5. GC-MS(EI) m/z: 279 (55%). 1H-NMR in d6-Aceton, ppm: 8,46 (s, 1H), 7,99 (s, 1H), 7,29 (1H).

Synthese von 2,2'-{(1-ethyl-1H-pyrrole-2,5-diyl)bis[(thieno[3,2-b]furan-2,5-diyl)methanylylidene]} dipropanedinitrile (A8) und 2,2'-{(1-phenyl-1H-pyrrole-2,5-diyl)bis[(thieno[3,2-b]furan-2,5-diyl) methanylylidene]}dipropanedinitrile (A9), allgemeine Vorschrift.

1 Äq von Bisstannylverbindung 6 oder 7 und 2 Äq von [(2-bromothieno[3,2-b]furan-5-yl)methylidene]propanedinitrile 5 wurden in Dioxan gelöst und die Lösung wurde entgast. 0,033 Äq von Bis-(tri-tert.-buthylphosphin)-palladium (0) wurden zugegeben und das Reaktionsgemisch wurde über Nacht auf 60°C erwärmt. Nachdem das Reaktionsgemisch auf Raumtemperatur abgekühlt war, wurde der ausgefallenen Niederschlag filtriert und mit Methanol gewaschen. Das Rohprodukt wurde aus Chlorbenzol umkristallisiert und anschließend sublimiert.
8: 33% Ausbeute, HPLC Reinheit 98,5 % @528 nm
9: 16% Ausbeute, HPLC-Reinheit 100% @574 nm

### Synthese Verbindung A13

### Synthese von 2,2'-{1H-indole-2,6-diylbis[(thieno[3,2-b]furan-2,5-diyl)methanylylidene]}dipropanedinitrile (A13)

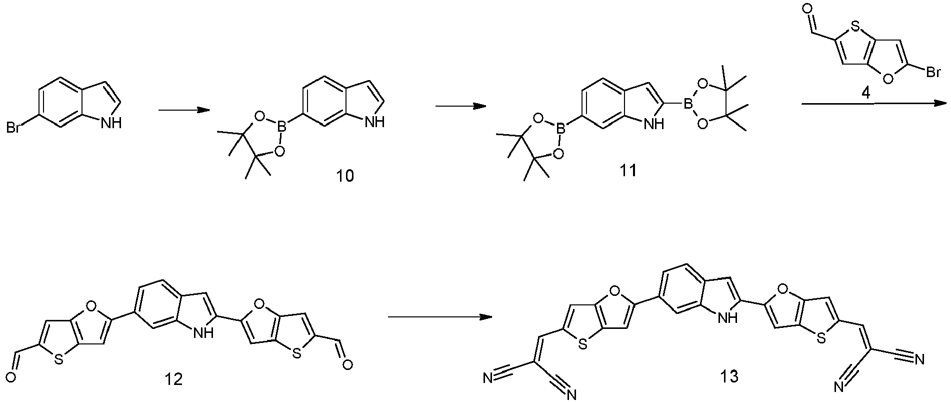

### Synthese von 6-(4,4,5,5-tetramethyl-1,3,2-dioxaborolan-2-yl)-1H-indole (10)

3,92 g (20 mmol) von 6-Bromindol wurden in 140 ml Dioxan gelöst. 5,59 g (22 mmol) von Bis-(pinakolato)-dibor und 5,89 g (60 mmol) von Kaliumacetat wurden zugegeben und Das Reaktionsgemisch wurde entgast. 293 mg (0,4 mmol) von 1,1'-Bis(diphenylphosphino)ferrocen-palladium(II)dichlorid wurden in das Reaktionsgemisch gegeben und es wurde über Nacht bei 90°C gerührt. Nachdem das Reaktionsgemisch auf Raumtemperatur abgekühlt war, wurde 2 molare Salzsäure-Lösung zugegeben. Es wurde mit Dichlormethan extrahiert, die organisch Phase würde mit ges. Ammoniumchlorid-Lösung, Wasser und ges. NaCl-Lösung gewaschen. Das Rohprodukt wurde durch Silikagelfiltration in Dichlormethan gereinigt, dabei wurden 1,49 g (31% Ausbeute) von 6-(4,4,5,5-tetramethyl-1,3,2-dioxaborolan-2-yl)-1H-indole **10** sauber isoliert. GC-MS (EI), m/z: 243 (100%), 228 (40%). 1H-NMR in d6-Aceton, ppm: 10,31 (sb, 1H), 7,89 (m, 1H), 7,57 (m, 1H), 7,43-7,40 (m, 2H), 6,48 (s, 1H), 1,21 (s, 12H).

### Synthese von 2,6-bis(4,4,5,5-tetramethyl-1,3,2-dioxaborolan-2-yl)-1H-indole (11)

0,78 g (3,07 mmol) von Bis-(pinakolato)-dibor, 60 mg (0,22 mmol) von 4,4-Di-tert-butyl-2,2-dipyridyl und 61 mg (0,09 mmol) von 1,5-Cyclooctadiene)(methoxy)iridium(I) dimer wurden in 31 ml Tetrahydrofuran gelöst. 1,49 g (6,14 mmol) von 6-(4,4,5,5-tetramethyl-1,3,2-dioxaborolan-2-yl)-1H-indole **10** wurde zum Reaktionsgemisch gegeben und es wurde bei 80°C über Nacht gerührt. Nachdem das Reaktionsgemisch auf Raumtemperatur abgekühlt war, Dichlormethan wurde zum Reaktionsgemisch gegeben und die organische Phase wurde mit Wasser und ges. NaCl-Lösung gewaschen. Das Rohproduckt wurde durch Säulenchromatographie an Silikagel mit 5% Aceton in Dichlormethan und anschließender Umkristallisation aus Ethanol gereinigt, dabei wurden 420 mg (18% Ausbeute) von 2,6-bis(4,4,5,5-tetramethyl-1,3,2-dioxaborolan-2-yl)-1H-indole 11 isoliert. 1H-NMR in d6-Aceton, ppm: 10,45 (s, 1H), 8,00 (s, 1H), 7,60 (d, 1H), 7,41 (d, 1H), 6,99 (s, 1H).

### Synthese von 2,2'-(1H-indole-2,6-diyl)di(thieno[3,2-b]furan-5-carbaldehyde) (12)

443 mg (1,2 mmol) von 2,6-bis(4,4,5,5-tetramethyl-1,3,2-dioxaborolan-2-yl)-1H-indole 11 wurden in 9 ml Ethanol gelöst. 566 mg (2,4 mmol) von 2-bromothieno[3,2-b]furan-5-carbaldehyde 4, 403 mg (4,8 mmol) Natriumhydrogencarbonat und Wasser wurde zum Reaktionsgemisch zugegeben und es wurde entgast. 65 mg (0,12 mmol) von Bis-(tri-tert.-buthylphosphin)-palladium (0) wurden zugegeben und das Reaktionsgemisch wurde über Nacht bei 80°C gerührt. Nachdem das Reaktionsgemisch auf Raumtemperatur abgekühlt war, wurde der ausgefallene Niederschlag filtriert und mit Methanol gewaschen. Das Rohprodukt wurde aus Toluol umkristallisiert, dabei wurden 280 mg (56% Ausbeute) von 2,2'-(1H-indole-2,6-diyl)di(thieno[3,2-b]furan-5-carbaldehyde) 12 isoliert. HPLC Reinheit: 85,8% @ 436 nm. 1H-NMR in d6-DMSO bei 80°C, ppm: 11,97 (sb, 1H), 9,92 (s, 1H), 9,91 (s, 1H), 8,18 (s, 1H), 8,16 (s, 1H), 7,91 (m, 1H), 7,74 (m, 1H), 7,61 (m, 1H), 7,53 (m, 2H), 7,08 (s, 1H).

### Synthese von 2,2'-{1H-indole-2,6-diylbis[(thieno[3,2-b]furan-2,5-diyl)methanylylidene]}dipropanedinitrile (A13)

280 mg (0,67 mmol) von 2,2'-(1H-indole-2,6-diyl)di(thieno[3,2-b]furan-5-carbaldehyde) 12 wurde in 7 ml Dimethylsulfoxid gelöst. 134 mg (2,01 mmol) von Malodinitril und 7,5 mg (0,07 mmol) von 1,4-Diazabicyclo[2,2,2]octan wurden zugegeben und das Reaktionsgemisch wurde über Nacht bei Raumtemperatur gerührt. Der ausgefallene Niederschlag wurde filtriert und mit Methanol gewaschen. Das Rohprodukt wurde aus Chlorbenzol umkristallisiert, dabei wurden 170 mg (49 % Ausbeute) von 2,2'-{1H-indole-2,6-diylbis[(thieno[3,2-b]furan-2,5iyl)methanylylidene]}dipropanedinitrile 13 isoliert. Weitere Aufreinigung erfolgte durch Sublimation.

### Synthese Verbindung A16

### Synthese von 2,2'-{1H-indole-4,7-diylbis[(thieno[3,2-b]furan-2,5-diyl)methanylylidene]}dipropanedinitrile (A16)

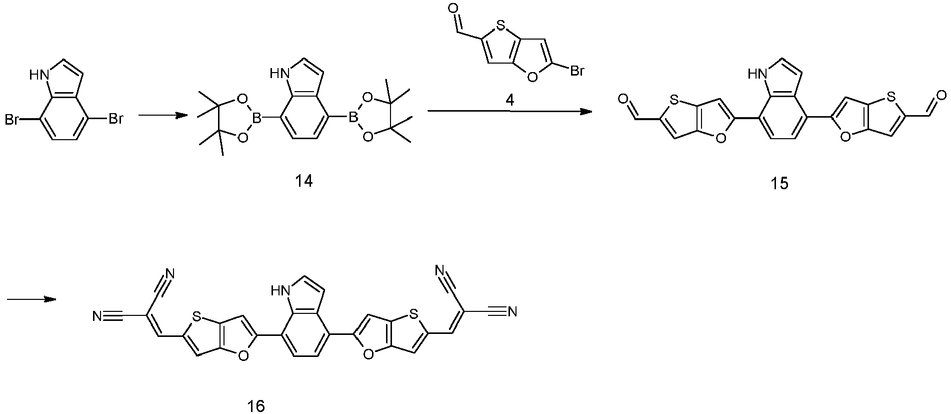

### Synthese von 4,7-bis(4,4,5,5-tetramethyl-1,3,2-dioxaborolan-2-yl)-1H-indole (14)

1,37 g (5 mmol) von 4,7-Dibromo-1H-indole wurde in 35 ml Dioxan gelöst. 2,79 g (11 mmol) von Bis-(pinakolato)-diboron und 2,94 g (30 mmol) von Kaliumacetat wurden zugegeben und es wurde entgast. 146 mg (0,2 mmol) von 1,1'-Bis(diphenylphosphino)ferrocen-palladium(II)dichlorid wurden zum Reaktionsgemisch gegeben und es wurde über Nacht bei 90°C gerührt. Nachdem das Reaktionsgemisch auf Raumtemperatur abgekühlt war, wurde 2 molare Salzsäure-Lösung zugegeben. Es wurde mit Dichlormethan extrahiert, die organisch Phase würde mit ges. Ammoniumchlorid-Lösung, Wasser und ges. NaCl-Lösung gewaschen. Das Rohprodukt wurde durch Silikagelfiltration in 50% Petrolether in Dichlormethan gereinigt, dabei wurden 1,34 g (73% Ausbeute) von 4,7-bis(4,4,5,5-tetramethyl-1,3,2-dioxaborolan-2-yl)-1H-indole 14 sauber isoliert. 1H-NMR in d6-Aceton, ppm: 9,94 (s, 1H), 7,54 (s, 2H), 7,36 (dd, 1H), 6,92 (dd, 1H), 1,38 (t, 24H).

### Synthese von 2,2'-(1H-indole-4,7-diyl)di(thieno[3,2-b]furan-5-carbaldehyde) (15)

1,17 g (3,17 mmol) von 4,7-bis(4,4,5,5-tetramethyl-1,3,2-dioxaborolan-2-yl)-1H-indole 14 wurde in 9 ml Dioxen gelöst. 1,46 g (6,37 mmol) 4, 5,38 g (25,4 mmol) Kaliumphosphat und 3 ml Wasser wurden zugegeben und das Reaktionsgemisch wurde entgast. 366 mg (0,32 mmol) Tetrakis-(triphenylphosphin)-palladium(0) wurde zugeben und es wurde über Nacht bei 80 °C gerührt. Nachdem das Reaktionsgemisch auf Raumtemperatur abgekühlt war, wurde der ausgefallene Niederschlag filtriert und mit Wasser und Methanol gewaschen. Das Rohprodukt wurde aus Toluol umkristallisiert, dabei wurden 434 mg (33% Ausbeute) von 2,2'-(1H-indole-4,7-diyl)di(thieno[3,2-b]furan-5-carbaldehyde) 15 isoliert. 1H-NMR in d6-Dimethylsulfoxidbei bei 100°C, ppm: 11,71 (sb, 1H), 9,98 (s, 1H), 9,93 (s, 1H), 8,29 (s, 1H), 8,22 (s, 1H), 7,83-7,80 (m, 3H), 7,78 (d, 1 H), 7,72 (dd, 1H), 7,17 (dd, 1H).

### Synthese von 2,2'-{1H-indole-4,7-diylbis[(thieno[3,2-b]furan-2,5-diyl)methanylylidene]}dipropanedinitrile (A16)

434 mg (1,04 mmol) von 2,2'-(1H-indole-4,7-diyl)di(thieno[3,2-b]furan-5-carbaldehyde) 15 wurden in 7 ml Dimethylsulfoxid gelöst. 694 mg (10,4 mmol) von Malodinitril und 12 mg (0,01 mmol) von 1,4-Diazabicyclo[2,2,2]octan wurden zugegeben und das Reaktionsgemisch wurde für 5 h gerührt. Der ausgefallene Niederschlag wurde filtriert und mit Methanol gewaschen. Das Rohprodukt wurde aus Chlorbenzol umkristallisiert und sublimiert, dabei wurden 132 mg (25% Ausbeute, HPLC Reinheit 99,5 % @ 541 nm) von 2,2'-{1H-indole-4,7-diylbis[(thieno[3,2-b]furan-2,5-diyl)methanylylidene]} dipropanedinitrile A16 isoliert.

### Synthese Gruppe 2:

### Synthese von Verbindung A25 und Verbindung A26

### Synthese von [(2-{5-[5-(2,2-dicyanoethenyl)furan-2-yl]-1-ethyl-1H-pyrrol-2-yl}thieno[3,2-b]furan-5-yl)methylidene]propanedinitrile (A25) und [(5-{5-[5-(2,2-dicyanoethenyl)thieno[3,2-b]furan-2-yl]-1H-pyrrol-2-yl}thiophen-2-yl)methylidene]propanedinitrile (A26)

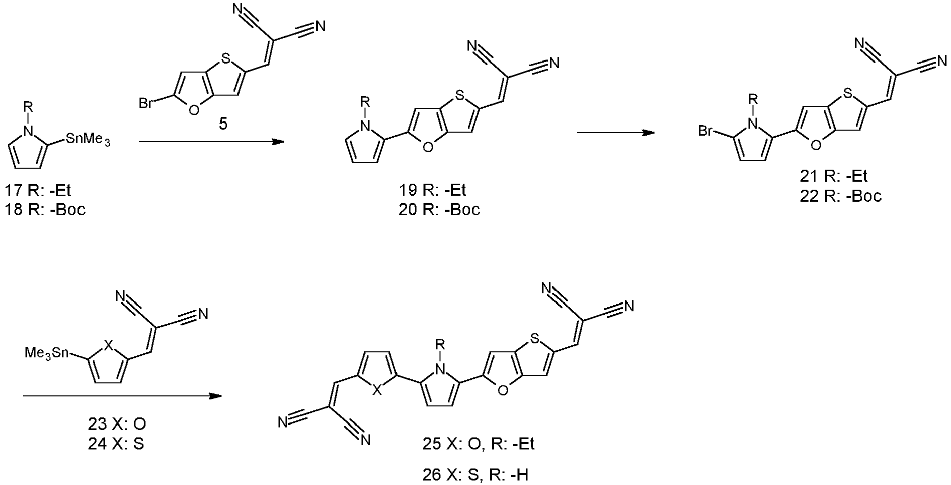

Verbindung 17 kann nach Tetrahedron, 71(33), 5399-5406; 2015 synthetisiert werden. Verbindung 18 kann nach Chemistry - A European Journal, 21(5), 2003-2010; 2015 synthetisiert werden. Verbindungen 23 und 24 können nach Eur. Pat. Appl., 3187496, 2017 synthetisiert werden.

### Synthese von {[2-(1-ethyl-1H-pyrrol-2-yl)thieno[3,2-b]furan-5-yl]methylidene}propanedinitrile (19)

910 mg (3,53 mmol) von 1-ethyl-2-(trimethylstannyl)1H-pyrrole 17 und 820 mg (2,94 mmol) von [(2-bromothieno[3,2-b]furan-5-yl)methylidene]propanedinitrile 5 wurden in 15 ml Dioxan gelöst und die Lösung wurde entgast. 37,6 mg (0.0735 mmol) von Bis-(tri-tert.-buthylphosphin)-palladium(0) wurden zugegeben und das Reaktionsgemisch wurde bei 80°C über Nacht gerührt. Nachdem das Reaktionsgemisch auf Raumtemperatur abgekühlt war, wurde der ausgefallene Niederschlag abfiltriert und mit Methanol gewaschen. Das Rohprodukt wurde durch Säulenchromatographie an Silikagel in Dichlormethan gereinigt, dabei wurde 439 mg (51% Ausbeute) von {[2-(1-ethyl-1H-pyrrol-2-yl)thieno[3,2-b]furan-5-yl]methylidene}propanedinitrile 19 isoliert. 1H-NMR in d6-Dimethylsulfoxid bei 90°C; ppm: 8,49 (s, 1H), 7,97 (s, 1H), 7,19 (s, 1H), 7,10 (dd, 1H), 6,74 (dd, 1H), 6,21 (dd, 1H), 4,23 (q, 2H), 1,36 (t, 3H). Die Synthese von Verbindung 20 erfolgte anlog aus Verbindung 18.

### Synthese von 2-bromo-5-[5-(2,2-dicyanoethenyl)thieno[3,2-b]furan-2-yl]-1H-pyrrol-1-yl (21)

513 mg (1,75 mmol) von {[2-(1-ethyl-1H-pyrrol-2-yl)thieno[3,2-b]furan-5-yl]methylidene}propanedinitrile 19 wurden in 30 ml DMF unter Argon gelöst. Zu dieser Lösung wurde bei -78°C eine Lösung aus 280 mg (1,57 mmol) von NBS in 5 ml DMF zugegeben und das Reaktionsgemisch wurde über Nacht bei -18°C gerührt. Das Reaktionsgemisch wurde auf Eis gegeben, Dichlormethan wurde zugegeben. Die wässrige Phase wurde mit Dichlormethan extrahiert. Das Rohprodukt wurde aus Ethanol umkristallisiert, es wurden 510 mg (78% Ausbeute) von 2-bromo-5-[5-(2,2-dicyanoethenyl)thieno[3,2-b]furan-2-yl]-1H-pyrrol-1-yl 21 isoliert. 1H-NMR in d6-Aceton, ppm: 8,37 (s, 1H), 7,98 (s, 1H), 7,25 (s, 1H), 6,81 (d, 1H), 6,36 (d, 1H), 4,39 (q, 2H), 1,4 (t, 3H). Die Synthese von Verbindung 22 erfolgte anlog aus Verbindung 20.

### Synthese von [(2-{5-[5-(2,2-dicyanoethenyl)furan-2-yl]-1-ethyl-1H-pyrrol-2-yl}thieno[3,2-b]furan-5-yl)methylidene]propanedinitrile (A25)

255 mg (0,685 mmol) von 2-bromo-5-[5-(2,2-dicyanoethenyl)thieno[3,2-b]furan-2-yl]-1H-pyrrol-1-yl 21 und 252 mg (0,822 mmol) von 5-(Trimethylstannyl)-2-dicyanovinylfuran 23 wurden in 3,5 ml Dioxan gelöst und es wurde entgast. Zum Reaktionsgemisch wurde 8,75 mg (0,017 mmol) von Bis-(tri-tert.-buthylphosphin)-palladium zugegeben und es wurde über Nacht bei 60°C gerührt. Nachdem das Reaktionsgemisch auf Raumtemperatur abgekühlt war, wurde der ausgefallene Niederschlag abfiltriert. Das Rohprodukt wurde mit Methanol gewaschen, und aus Toluol und Chlorbenzol umkristallisiert. Anschließend wurde das Produkt sublimiert, dabei wurden 114 mg (Ausbeute 38%, HPLC-Reinheit 99,7% @542 nm) von [(2-{5-[5-(2,2-dicyanoethenyl)furan-2-yl]-1-ethyl-1H-pyrrol-2-yl}thieno[3,2-b]furan-5-yl)methylidene]propanedinitrile A25 isoliert. Die Synthese von Verbindung A26 erfolgte anlog aus Verbindungen 22 und 24.

### Synthese von Verbindung A37 und Verbindung A38

### Synthese von {[3-chloro-5-(5-{5-[5-(2,2-dicyanoethenyl)thieno[3,2-b]furan-2-yl]-1-ethyl-1H-pyrrol-2-yl}furan-2-yl)thiophen-2-yl]methylidene}propanedinitrile (A37) und {[2-(5-{5-[5-(2,2-dicyanoethenyl)-1,3-thiazol-2-yl]furan-2-yl}-1-ethyl-1H-pyrrol-2-yl)thieno[3,2-b]furan-5-yl]methylidene}propanedinitrile (A38)

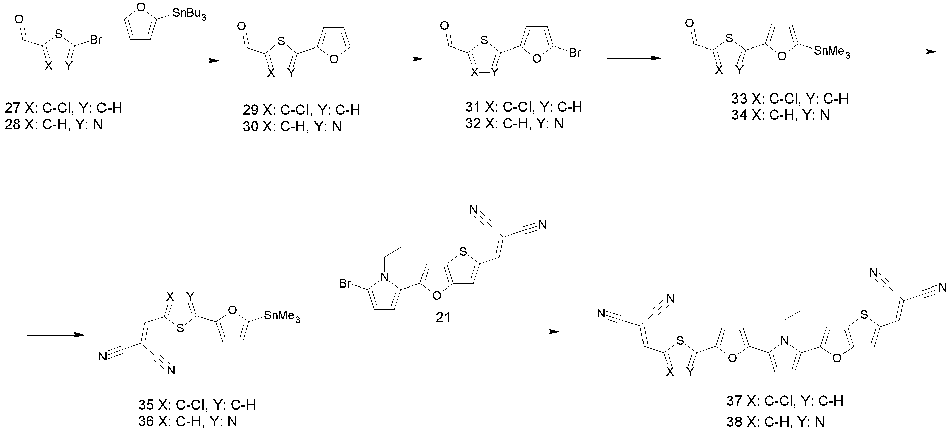

### Synthese von 3-chloro-5-(furan-2-yl)thiophene-2-carbaldehyde (29)

7,27 g (19,8 mmol) 2-(Tributhylstannyl)furan wurde in 99 ml Dioxan gelöst. Es wurden 5 g (21,7 mmol) 5-Brom-3-chlorthiophen-2-carbaldehyd zugegeben und die Lösung wurde entgast. 252 mg (0,49 mmol) Bis-(tri-tert.-buthylphosphin)-palladium (0) wurde zum Reaktionsgemisch gegeben und es wurde für 4 h bei 60°C gerührt. Nachdem das Reaktionsgemisch auf Raumtemperatur abgekühlt war, wurde Dichlormethan zugegeben. Die organische Phase wurde mit Wasser und ges. NaCl-Lösung gewaschen. Das Rohprodukt wurde durch Silikagelfiltration in Dichlormethan gereinigt, dabei wurden 10,8 g (110% Ausbeute, Reinheit 43%) 3-chloro-5-(furan-2-yl)thiophene-2-carbaldehyde 29 isoliert. 1H-NMR in d6-Aceton, ppm: 10,01 (s, 1H), 7,77 (dd, 1H), 7,48 (s, 1H), 7,12 (dd, 1H), 6,67 (dd, 1H). Die Verbindung 30 kann analog aus 2-bromo-1,3-thiazole-5-carbaldehyde synthetisiert werden.

### Synthese von 5-(5-bromofuran-2-yl)-3-chlorothiophene-2-carbaldehyde (31)

10,8 g (21,8 mmol) 3-chloro-5-(furan-2-yl)thiophene-2-carbaldehyde 29 wurden in 50 ml N,N-Dimethylformamid gelöst. 3,96 g (21,8 mmol) N-Bromsuccinimid wurden in kleinen Portionen bei 0°C zugegeben und das Reaktionsgemisch wurde über Nacht bei Raumtemperatur gerührt. Das Reaktionsgemisch wurde auf Eis gegeben, der ausgefallene Niederschlag wurde filtriert und mit Wasser gewaschen. Das Rohprodukt wurde an der Luft getrocknet und mittels Säulenchromatographie an Silikagel in 50% Petrolether in Dichlormethan gereinigt. Dabei wurden 4,36 g (69% Ausbeute) 5-(5-bromofuran-2-yl)-3-chlorothiophene-2-carbaldehyde 31 sauber isoliert. 1H-NMR in d6-Aceton, ppm: 10,02 (s, 1h), 7,51 (s, 1H), 7,15 (d, 1H), 6,73 (d, 1H). Die Verbindung 32 kann analog synthetisiert werden.

### Synthese von 3-chloro-5-[5-(trimethylstannyl)furan-2-yl]thiophene-2-carbaldehyde (33)

1,8 ml (16,4 mmol) 1-Methylpiperazin wurden in 75 ml Tetrahydrofuran gelöst. Bei -78 °C wurden 6,5 ml (16,4 mmol) 2,5 N n-Buthyllithium wurden tropenweise zugegeben. 4,34 g (14,9 mmol) 5-(5-bromofuran-2-yl)-3-chlorothiophene-2-carbaldehyde 31 wurden in 64 ml Tetrahydrofuran gelöst und tropfenweise in die Reaktionslösung bei - 78°C zugegeben. 2,73 ml (17,9 mmol) N,N,N',N'-Tetramethylethylendiamin wurden zu der Reaktionslösung gegeben. Anschließend wurden 7,15 ml (19,9 mmol) 2,5 N n-Buthyllithium in das Reaktionsgemsch gegeben und es wurde für 1 h bei -78°C gerührt. 17,9 ml (17,9 mmol) Trimethylstannylchlorid wurden in das Reaktionsgemisch gegeben und es wurde über Nacht auf Raumtemperatur erwärmt. Es wurde ges. NaCl-Lösung und Methyl-tert-butylether zum Reaktionsgemisch gegeben. Die organische Phase wurde mit Wasser und ges. NaCl-Lösung gewaschen. Das Rohprodukt 5,5 g (98,3% Ausbeute) 3-chloro-5-[5-(trimethylstannyl)furan-2-yl]thiophene-2-carbaldehyde 33 wurde isoliert und ohne Aufreinigung umgesetzt.

Die Verbindung 34 kann analog synthetisiert werden.

### Synthese von ({3-chloro-5-[5-(trimethylstannyl)furan-2-yl]thiophen-2-yl}methylidene)propanedinitrile (35)

5,48 g (14,6 mmol) 3-chloro-5-[5-(trimethylstannyl)furan-2-yl]thiophene-2-carbaldehyde 33 wurden in 14 ml Ethanol gelöst. 1,16 g (17,5 mmol) Malodinitril und 119 mg (1,31 mmol) β-Alanin wurden zugegeben und das Reaktionsgemisch wurde über Nacht bei Raumtemperatur gerührt. Der ausgefallene Niederschlag wurde abfiltriert und mit Ethanol gewaschen, dabei wurden 2,1 g (34% Ausbeute) von ({3-chloro-5-[5-(trimethylstannyl)furan-2-yl]thiophen-2-yl}methylidene)propanedinitrile A35 isoliert, welches ohne weiter Aufreinigung umgesetzt wurde. 1H-NMR in d6-Aceton, ppm: 8,30 (s, 1H), 7,63 (s, 1H), 7,26 (d, 1H), 6,91 (d, 1H), 0,41 (s, 9H). Die Verbindung 36 kann analog synthetisiert werden.

### Synthese von {[3-chloro-5-(5-{5-[5-(2,2-dicyanoethenyl)thieno[3,2-b]furan-2-yl]-1-ethyl-1H-pyrrol-2-yl}furan-2-yl)thiophen-2-yl]methylidene}propanedinitrile (A37)

457 mg (1.23 mmol) {[2-(5-bromo-1-ethyl-1H-pyrrol-2-yl)thieno[3,2-b]furan-5-yl]methylidene}propanedinitrile 21 und 624 mg (1,47 mmol) ({3-chloro-5-[5-(trimethylstannyl)furan-2-yl]thiophen-2-yl}methylidene)propanedinitrile 35 wurden in 2,5 ml Dioxan gelöst und entgast. 15,7 mg (0,03 mmol) Bis-(tri-tert.-buthylphosphin)-palladium (0) wurden zugegeben und das Reaktionsgemisch wurde über Nacht bei 60°C gerührt. Nachdem das Reaktionsgemisch auf Raumtemperatur abgekühlt war, wurde der ausgefallene Niederschlag filtriert und mit Methanol gewaschen. Das Rohprodukt wurde aus Toluol umkristallisiert und sublimiert, dabei wurden 37 mg (5,5% Ausbeute, HPLC-Reinheit 98,0% @ 567 nm) von {[3-chloro-5-(5-{5-[5-(2,2-dicyanoethenyl)thieno[3,2-b]furan-2-yl]-1-ethyl-1H-pyrrol-2-yl}furan-2-yl)thiophen-2-yl]methylidene}propanedinitrile A37 isoliert. Die Verbindung 38 kann analog synthetisiert werden.

## Patentansprüche

1. Verbindung der allgemeinen Formel I
A1-M-(T1)ₐ-(T2)_{b}-(T3)_{c}-A2 (I)
mit den Parametern a, b, c jeweils unabhängig voneinander 0, 1 oder 2, mit der Maßgabe, dass mindestens einer der Parameter a, b, c = 1 oder 2 ist,
mit der Gruppe M ausgewählt aus der Gruppe bestehend aus: 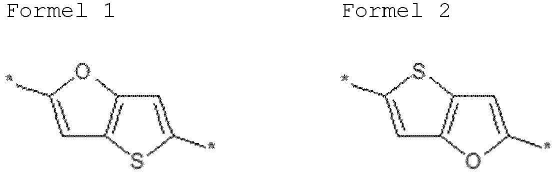 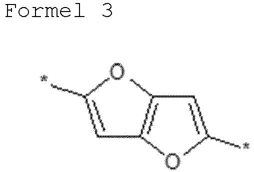
wobei * die Anknüpfung an die Gruppen T1, T2, T3, A1 und A2 bezeichnet, wobei H-Atome der Formeln 1 bis 4 durch Alkyl, O-Alkyl, S-Alkyl, oder Halogen substituiert sein können;
mit den Gruppen T1, T2 und T3 unabhängig voneinander ausgewählt aus der Gruppe bestehend aus: 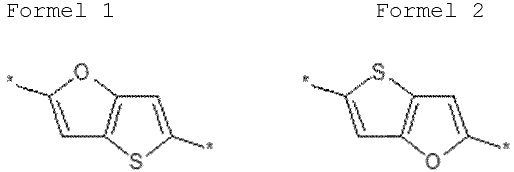 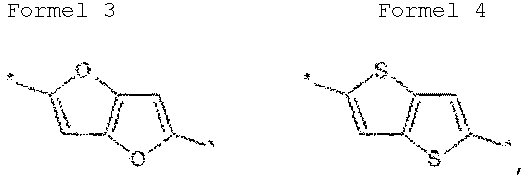
wobei H-Atome der Formeln 1 bis 4 durch Alkyl, O-Alkyl, S-Alkyl, oder Halogen substituiert sein können, 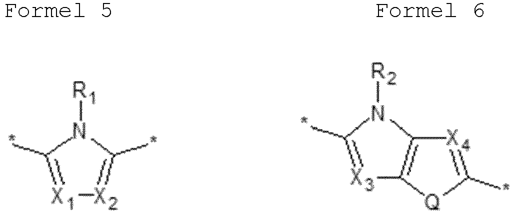 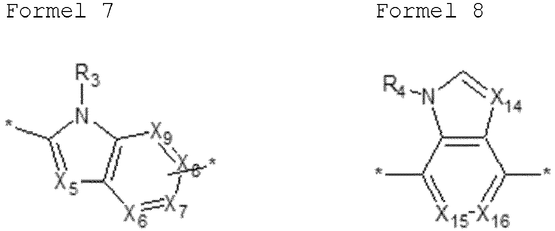
mit R1 bis R4 jeweils unabhängig voneinander ausgewählt aus der Gruppe bestehend aus H, Alkyl, Alkenyl, Aryl und Heteroaryl; 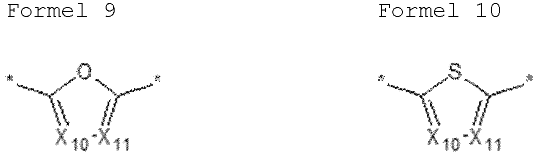 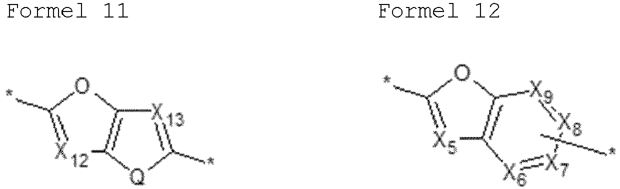 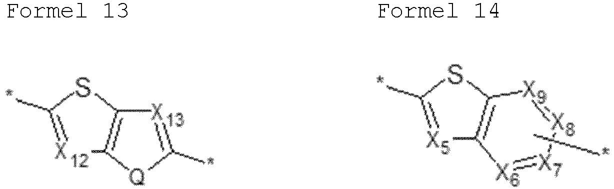 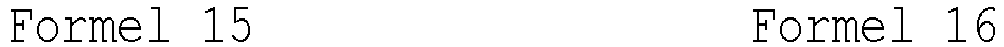 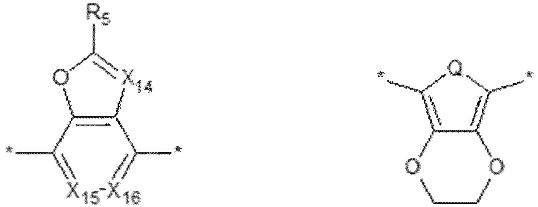 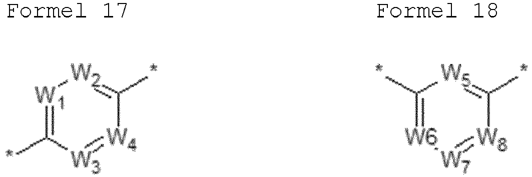
mit X₁ bis X₁₆ unabhängig voneinander ausgewählt aus N oder CR₆, mit der Maßgabe, dass in den Formeln 7, 12 und 14 jeweils eine Gruppe aus X₆ bis X₉ die Anknüpfung * an eine weitere Gruppe in der Verbindung der allgemeinen Formel I bezeichnet,
mit Q ausgewählt aus der Gruppe bestehend aus O, S, und NR₇,
mit W₁ bis W₈ jeweils unabhängig voneinander ausgewählt aus N und CR₈, wobei R₆, R₇ und R₈ jeweils unabhängig voneinander ausgewählt sind aus der Gruppe bestehend aus H, CN, Alkyl, Alkenyl, O-Alkyl, S-Alkyl, Aryl, und Heteroaryl;
wobei die elektronenziehenden Gruppen A1 und A2 unabhängig voneinander elektronenziehende Gruppen mit zumindest einer C=C-Doppelbindung sind, und wobei * die Anknüpfung an eine weitere Gruppe M, T1, T2, T3, und A2 in der Verbindung der allgemeinen Formel I bezeichnet.

2. Verbindung nach Anspruch 1, wobei T1 unabhängig voneinander ausgewählt ist aus der Gruppe bestehend aus: 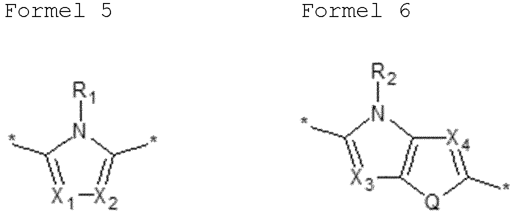 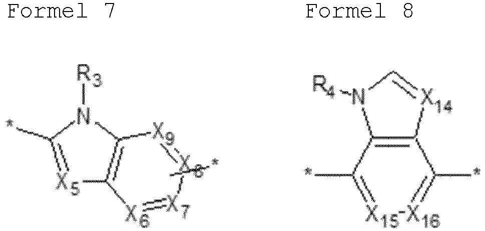 wobei bevorzugt X₁ und X₂ und/oder X₃ und X₄ CR₆ sind, bevorzugt ist R₆ ausgewählt aus H und Alkyl; und/oder wobei bevorzugt X₅ CR₆ mit R₆ H oder Alkyl ist, und/oder wobei bevorzugt X₆ bis X₉ CR₆ ist, und/oder wobei bevorzugt R₁ bis R₃ unabhängig voneinander H, Alkyl oder Aryl sind, und/oder wobei bevorzugt R₄ H oder Alkyl ist.

3. Verbindung nach Anspruch 1 oder 2, wobei T2 und T3 ausgewählt sind aus der Gruppe bestehend aus: 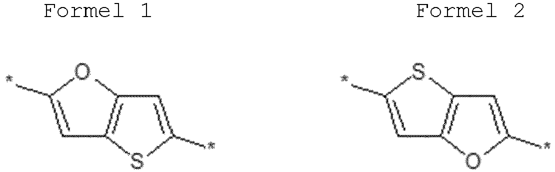 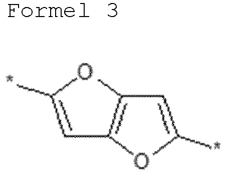 wobei H-Atome der Formeln 1 bis 4 durch Alkyl, O-Alkyl, S-Alkyl, oder Halogen substituiert sein können, bevorzugt sind H-Atome der Formeln 1 bis 4 nicht substituiert, 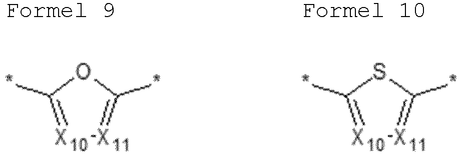 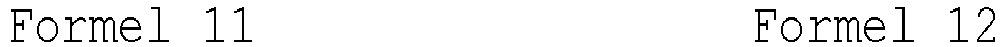 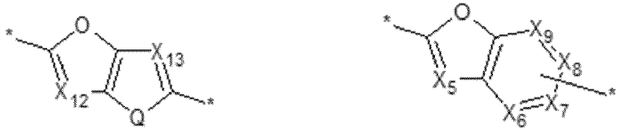 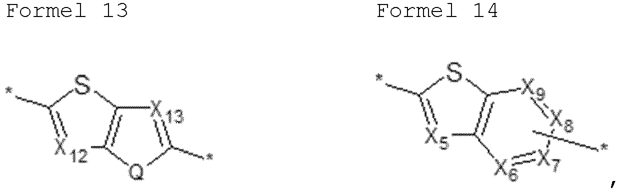 wobei bevorzugt R₆ und R₇ jeweils unabhängig voneinander ausgewählt sind aus der Gruppe bestehend aus H, Alkyl, und Aryl.

4. Verbindung nach einem der vorhergehenden Ansprüche, wobei A1 ausgewählt ist aus der Gruppe bestehend aus: 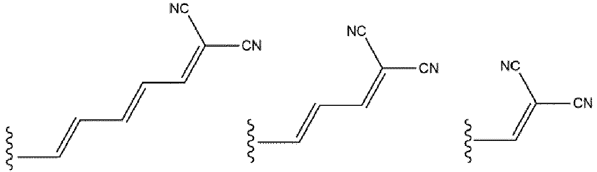 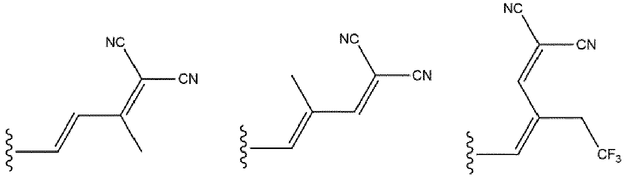 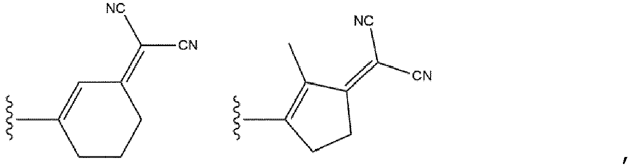 und wobei A2 ausgewählt ist aus der Gruppe bestehend aus:

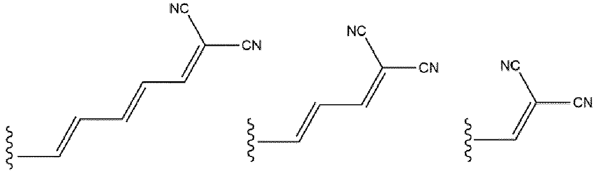

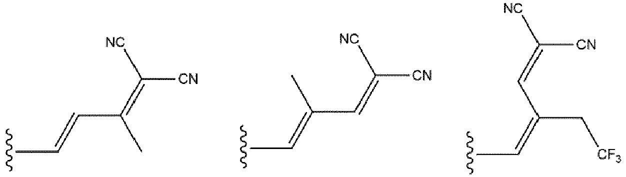

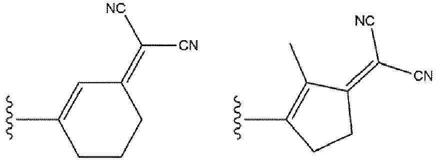

5. Verbindung nach einem der vorhergehenden Ansprüche, wobei die Parameter a, b, c bevorzugt jeweils unabhängig voneinander 0 oder 1 sind, mit der Maßgabe, dass mindestens einer der Parameter a, b, c gleich 1 ist, bevorzugt sind mindestens zwei der Parameter a, b, c gleich 1; oder wobei der Parameter a gleich 1 oder 2, mit mindestens einem der Parameter b und c gleich 1 oder 2, bevorzugt mit einem der Parameter b oder c gleich 1 oder 2, bevorzugt mit einem der Parameter b oder c gleich 1, insbesondere bevorzugt mit dem Parameter c gleich 1 oder 2.

6. Verbindung nach einem der vorhergehenden Ansprüche,
wobei die Gruppe M ausgewählt ist aus der Gruppe bestehend aus: 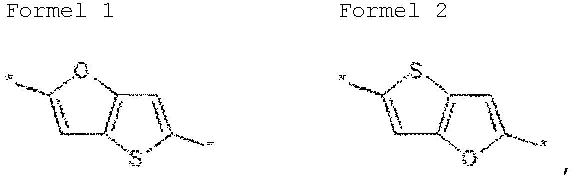 und
wobei T1 ausgewählt ist aus der Gruppe bestehend aus 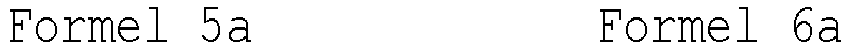 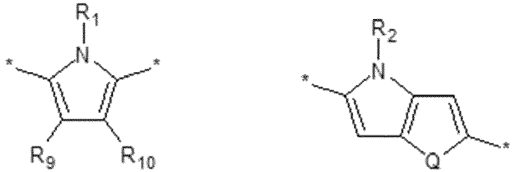 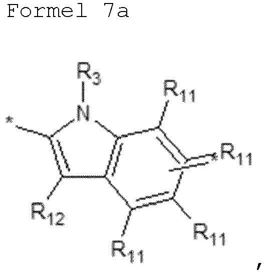
wobei R₉ und R₁₀ jeweils unabhängig voneinander ausgewählt sind aus der Gruppe bestehend aus H, Halogen, bevorzugt F und Cl, CN, Alkyl, Alkenyl, O-Alkyl, S-Alkyl, Aryl, Heteroaryl, bevorzugt sind R₉ und R₁₀ unabhängig voneinander H oder Alkyl,
wobei R₁₁ und R₁₂ jeweils unabhängig voneinander ausgewählt sind aus der Gruppe bestehend aus H, Halogen, bevorzugt F und Cl, CN, Alkyl, Alkenyl, O-Alkyl, S-Alkyl, Aryl, Heteroaryl, bevorzugt sind R₁₁ und R₁₂ H.

7. Verbindung nach einem der vorhergehenden Ansprüche, wobei die Verbindung die allgemeinen Formel II aufweist
A1-M-(T1)ₐ-(T3)_{c}-A2 (II),
wobei bevorzugt a und c gleich 1 sind, a gleich 1 und c gleich 2 sind, oder a gleich 2 und c gleich 1 sind.

8. Verbindung nach einem der vorhergehenden Ansprüche, wobei die Verbindung ausgewählt ist aus der Gruppe bestehend aus:

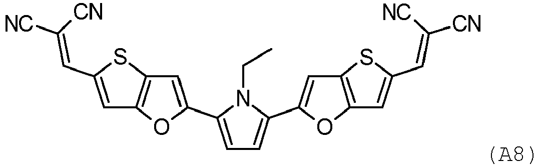

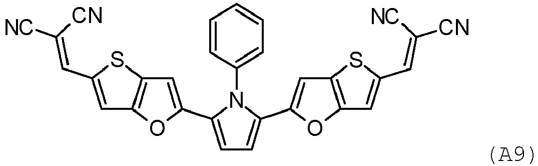

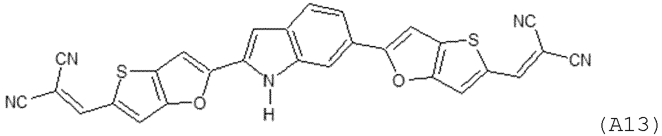

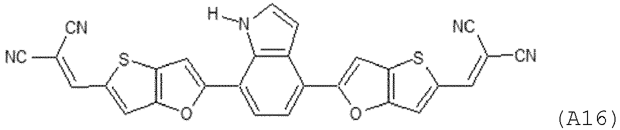

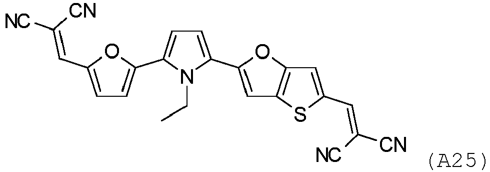

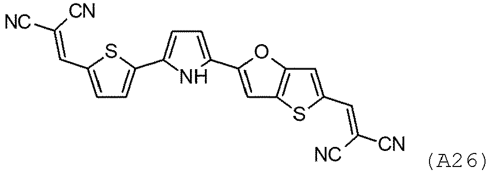

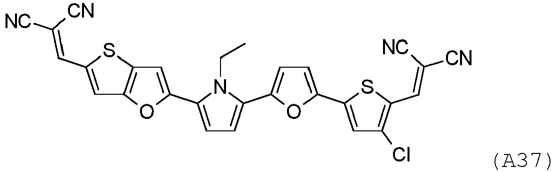

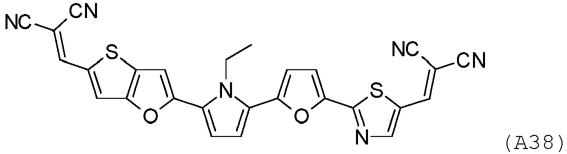

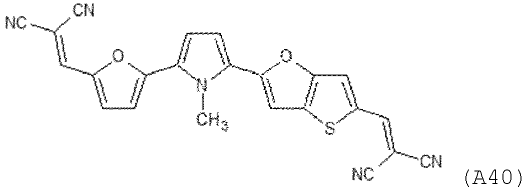

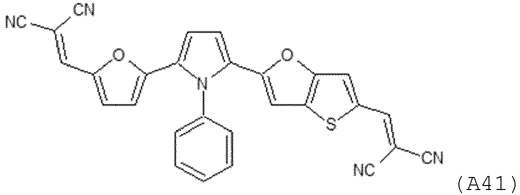

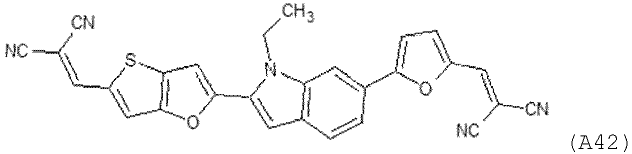

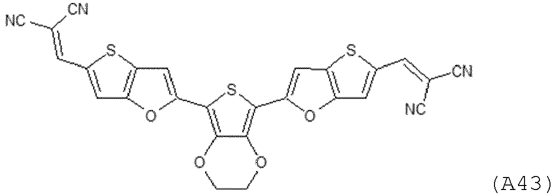

9. Verwendung mindestens einer Verbindung nach einem der Ansprüche 1 bis 8 in einem organischen elektronischen Bauelement, bevorzugt in einem organischen photovoltaischen Element.

10. Organisches elektronisches Bauelement umfassend eine Elektrode, eine Gegenelektrode und mindestens eine organische photoaktive Schicht zwischen der Elektrode und der Gegenelektrode, wobei die mindestens eine organische photoaktive Schicht mindestens eine Verbindung nach einem der Ansprüche 1 bis 8 aufweist.

## Claims

1. Compound of the general formula I
A1-M-(T1)ₐ-(T2)_{b}-(T3)_{c}-A2 (I)
wherein the parameters a, b and c are each independently of one another 0, 1 or 2, with the proviso that at least one of parameters a, b, c is equal to 1 or 2,
wherein the group M is selected from the group consisting of: 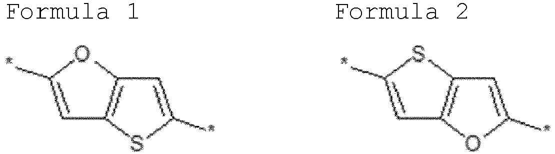 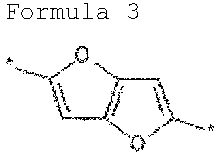
wherein * denotes the linkage to the groups T1, T2, T3, A1 and A2, wherein hydrogen atoms in formulas 1 to 4 may be substituted by alkyl, O-alkyl, S-alkyl or halogen; wherein the groups T1, T2 and T3 are independently of one another selected from the group consisting of: 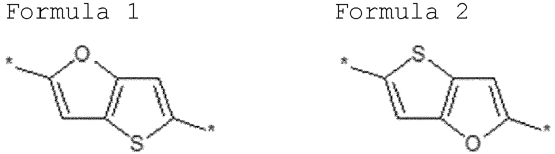 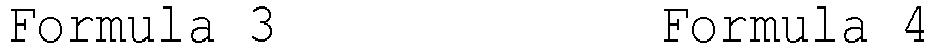 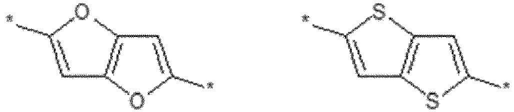
wherein hydrogen atoms in formulas 1 to 4 may be substituted by alkyl, O-alkyl, S-alkyl or halogen, 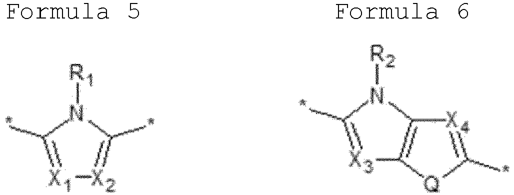 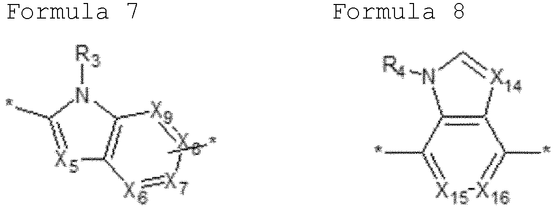
wherein R1 to R4 are each independently of one another selected from the group consisting of H, alkyl, alkenyl, aryl and heteroaryl; 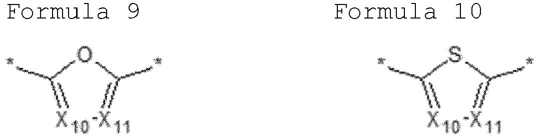 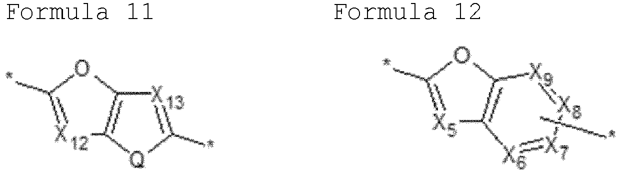 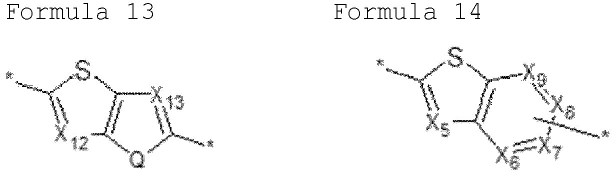 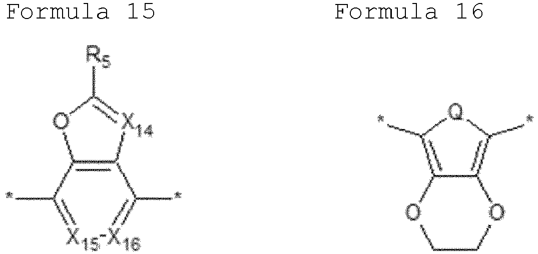 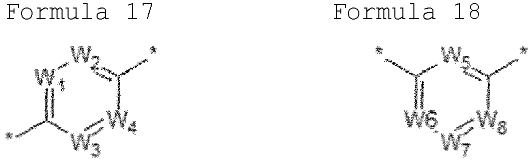
wherein X₁ to X₁₆ are independently of one another selected from N or CR₆, with the proviso that in formulas 7, 12 and 14 in each case one group out of X₆ to X₉ denotes the linkage * to a further group in the compound of the general formula I,
wherein Q is selected from the group consisting of O, S and NR₇,
wherein W₁ to W₈ are each independently of one another selected from N and CR₈, wherein R₆, R₇ and R₈ are each independently of one another selected from the group consisting of H, CN, alkyl, alkenyl, O-alkyl, S-alkyl, aryl and heteroaryl;
wherein the electron-withdrawing groups A1 and A2 are independently of one another electron-withdrawing groups having at least one C=C double bond, and wherein * denotes the linkage to a further group M, T1, T2, T3 or A2 in the compound of the general formula I.

2. Compound according to Claim 1, wherein T1 is independently of one another selected from the group consisting of: 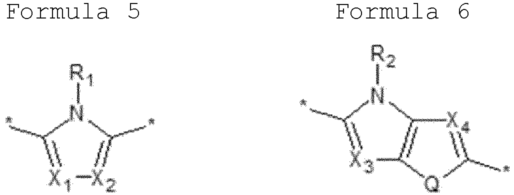 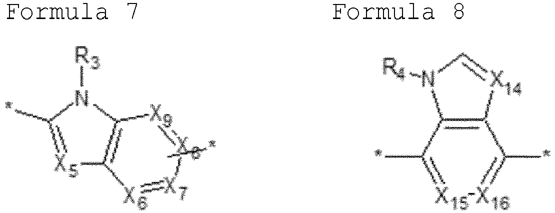 wherein preferably X₁ and X₂ and/or X₃ and X₄ are CR₆, preferably R₆ is selected from H and alkyl; and/or wherein preferably X₅ is CR₆ wherein R₆ is H or alkyl, and/or wherein preferably X₆ to X₉ is CR₆, and/or wherein preferably R₁ to R₃ are independently of one another H, alkyl or aryl, and/or wherein preferably R₄ is H or alkyl.

3. Compound according to Claim 1 or 2, wherein T2 and T3 are selected from the group consisting of: 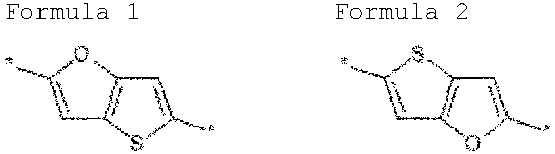 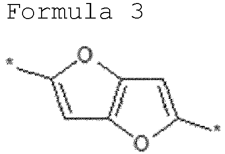
wherein hydrogen atoms in formulas 1 to 4 may be substituted by alkyl, O-alkyl, S-alkyl or halogen, preferably hydrogen atoms in formulas 1 to 4 are unsubstituted, 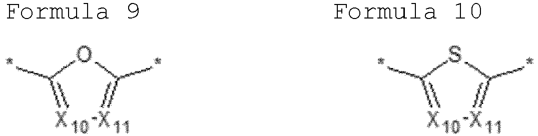 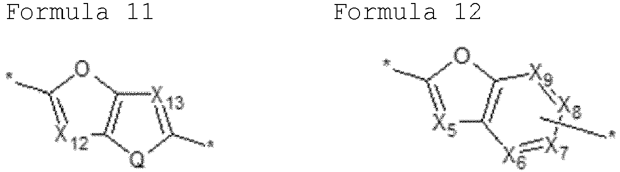 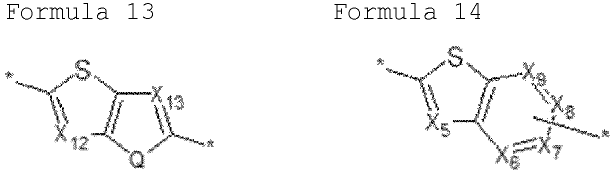
wherein preferably R₆ and R₇ are each independently of one another selected from the group consisting of H, alkyl and aryl.

4. Compound according any of the preceding claims, wherein A1 is selected from the group consisting of: 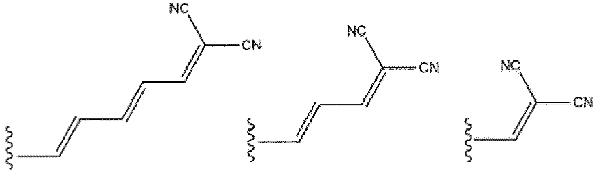 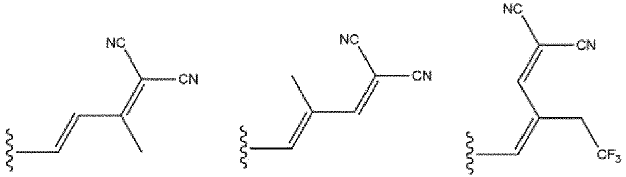 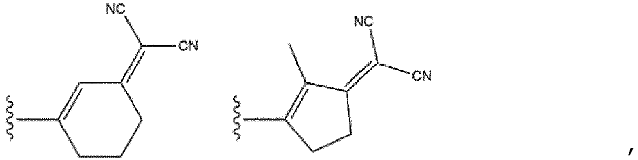 and wherein A2 is selected from the group consisting of:

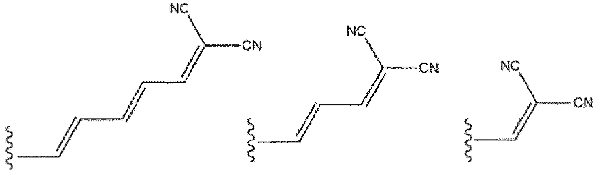

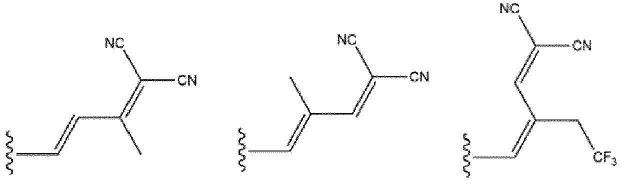

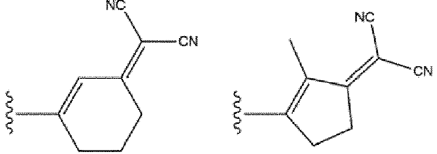

5. Compound according any of the preceding claims, wherein the parameters a, b and c are preferably each independently of one another 0 or 1, with the proviso that at least one of parameters a, b, c is equal to 1, preferably at least two of parameters a, b, c is equal to 1; or wherein the parameter a is equal to 1 or 2, wherein at least one of parameters b and c is equal to 1 or 2, preferably wherein one of parameters b or c is equal to 1 or 2, preferably wherein one of parameters b or c is equal to 1, more preferably wherein parameter c is equal to 1 or 2.

6. Compound according any of the preceding claims, wherein the group M is selected from the group consisting of: 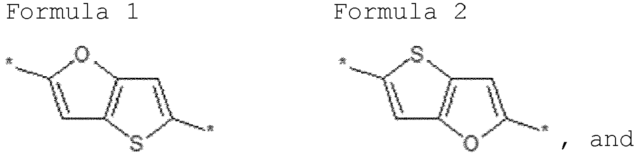 wherein T1 is selected from the group consisting of 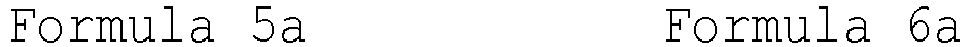 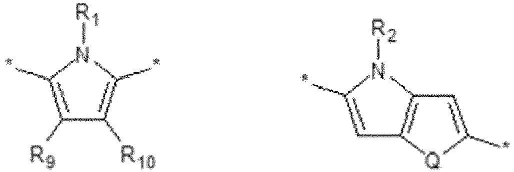 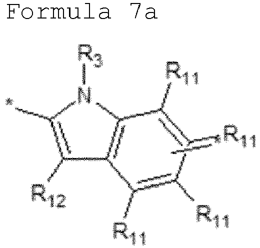
wherein R₉ and R₁₀ are each independently of one another selected from the group consisting of H, halogen, preferably F or Cl, CN, alkyl, alkenyl, O-alkyl, S-alkyl, aryl, heteroaryl, preferably R₉ and R₁₀ are independently of one another H or alkyl,
wherein R₁₁ and R₁₂ are each independently of one another selected from the group consisting of H, halogen, preferably F or Cl, CN, alkyl, alkenyl, O-alkyl, S-alkyl, aryl, heteroaryl, preferably R₁₁ and R₁₂ are H.

7. Compound according any of the preceding claims, wherein the compound has the general formula II
A1-M-(T1)ₐ-(T3)_{c}-A2 (II)
wherein preferably a and c are equal to 1, a is equal to 1 and c is equal to 2, or a is equal to 2 and c is equal to 1.

8. Compound according any of the preceding claims, wherein the compound is selected from the group consisting of:

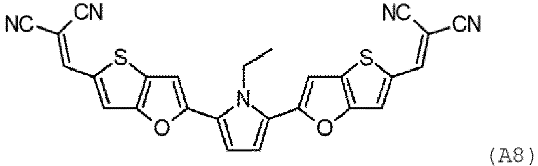

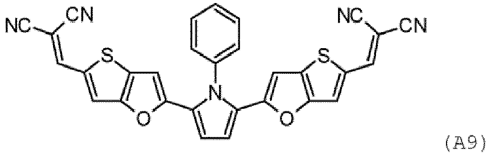

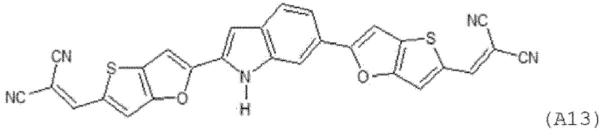

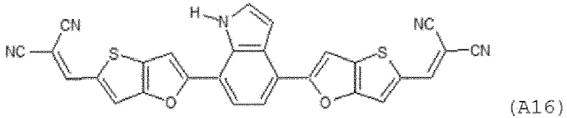

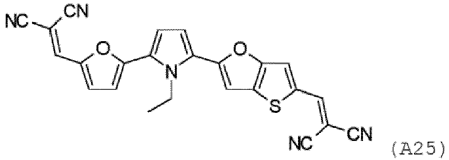

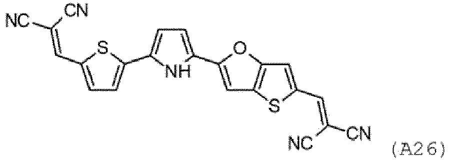

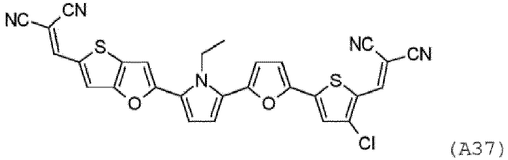

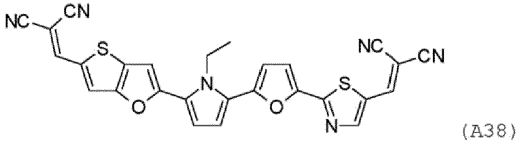

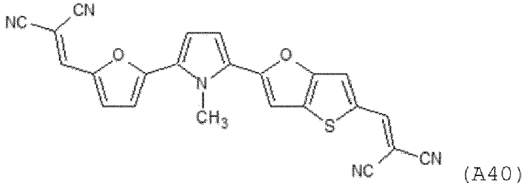

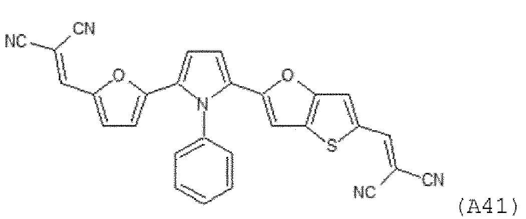

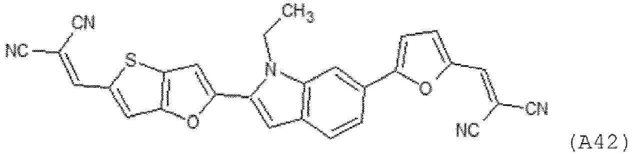

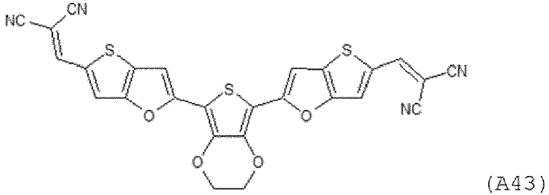

9. Use of at least one compound according any of Claims 1 to 8 in an organic electronic component, preferably in an organic photovoltaic element.

10. Organic electronic component comprising an electrode 2, a counter electrode 6 and at least one organic photoactive layer 4 between the electrode and the counter electrode, wherein the at least one organic photoactive layer includes at least one compound according to any of Claims 1 to 8.

## Revendications

1. Composé de formule générale I
A1-M-(T1)ₐ-(T2)_{b}-(T3)_{c}-A2 (I)
les paramètres a, b, c représentant, à chaque fois indépendamment les uns des autres, 0, 1 ou 2, à condition qu'au moins l'un des paramètres a, b, c = 1 ou 2, le groupe M étant choisi dans le groupe constitué par : 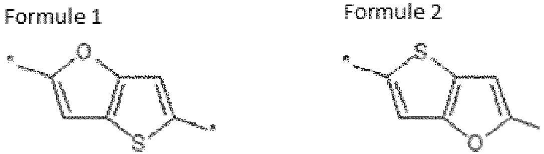 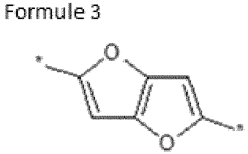
* désignant la liaison aux groupes T1, T2, T3, A1 et A2, les atomes de H des formules 1 à 4 pouvant être substitués par alkyle, O-alkyle, S-alkyle ou halogène ;
les groupes T1, T2 et T3 étant choisis, indépendamment les uns des autres, dans le groupe constitué par : 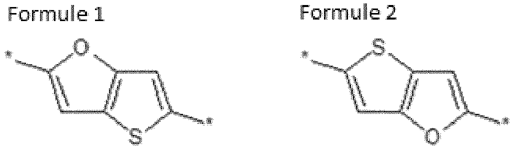 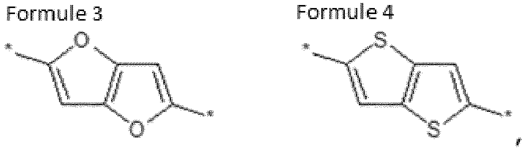
les atomes de H des formules 1 à 4 pouvant être substitués par alkyle, O-alkyle, S-alkyle ou halogène, 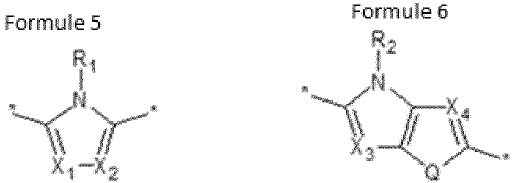 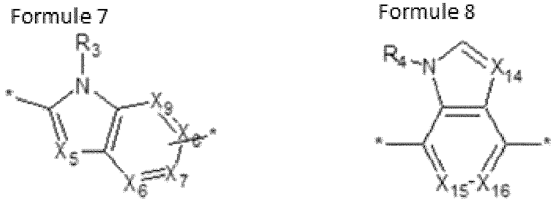
R₁ à R₄ étant choisis, à chaque fois indépendamment les uns des autres, dans le groupe constitué par H, alkyle, alcényle, aryle et hétéroaryle ; 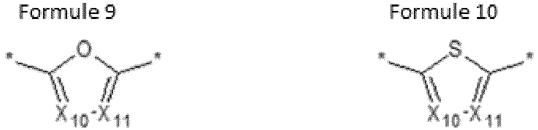 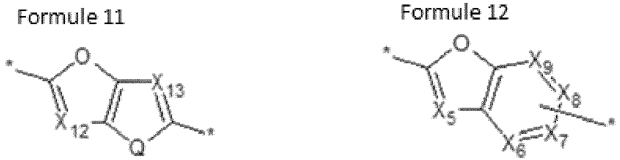 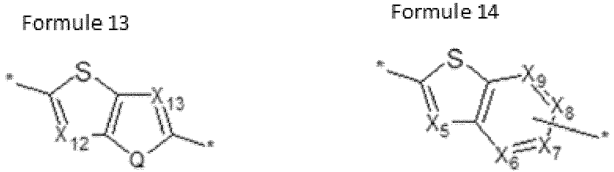 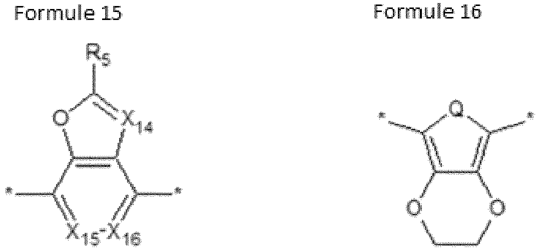 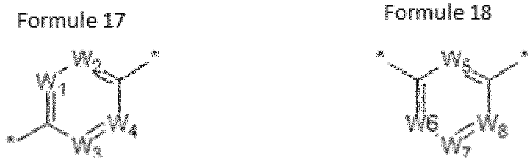
X₁ à X₁₆ étant choisis, indépendamment les uns des autres, parmi N ou CR₆, à condition que dans les formules 7, 12 et 14, à chaque fois un groupe parmi X₆ à X₉ désigne la liaison * à un autre groupe dans le composé de formule générale I,
Q étant choisi dans le groupe constitué par O, S et NR₇ ; W₁ à W₈ étant choisis, à chaque fois indépendamment les uns des autres, parmi N et CR₈; R₆, R₇ et R₈ étant choisis, à chaque fois indépendamment les uns des autres, dans le groupe constitué par H, CN, alkyle, alcényle, O-alkyle, S-alkyle, aryle et hétéroaryle ;
les groupes attracteurs d'électrons A1 et A2 représentant, indépendamment l'un de l'autre, des groupes attracteurs d'électrons présentant au moins une double liaison C=C et * désignant la liaison à un autre groupe M, T1, T2, T3 et A2 dans le composé de formule générale I.

2. Composé selon la revendication 1, T1 étant choisi, indépendamment, dans le groupe constitué par : 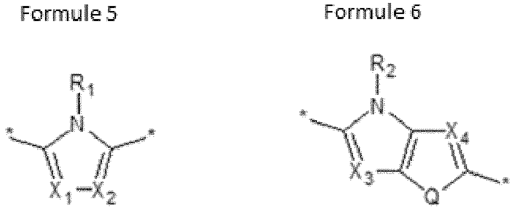 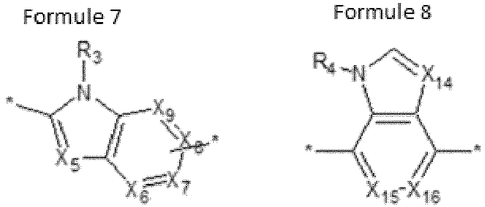 X₁ et X₂ et/ou X₃ et X₄ représentant de préférence CR₆, R₆ étant de préférence choisi parmi H et alkyle ; et/ou X₅ représentant de préférence CR₆, R₆ représentant H ou alkyle ; et/ou X₆ à X₉ représentant de préférence CR₆; et/ou R₁ à R₃ représentant de préférence, indépendamment les uns des autres, H, alkyle ou aryle et/ou R₄ représentant de préférence H ou alkyle.

3. Composé selon la revendication 1 ou 2, T2 et T3 étant choisis dans le groupe constitué par : 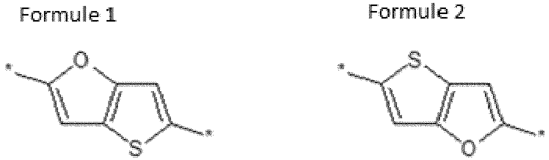 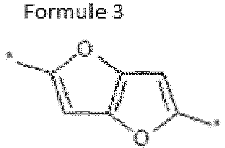 les atomes de H des formules 1 à 4 pouvant être substitués par alkyle, O-alkyle, S-alkyle ou halogène, de préférence les atomes de H des formules 1 à 4 n'étant pas substitués, 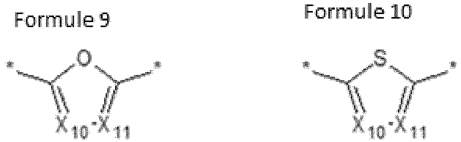 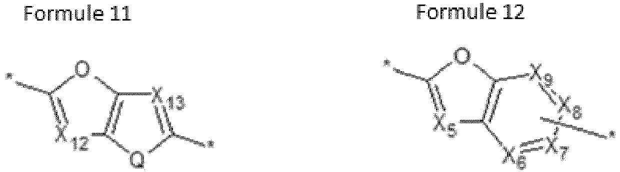 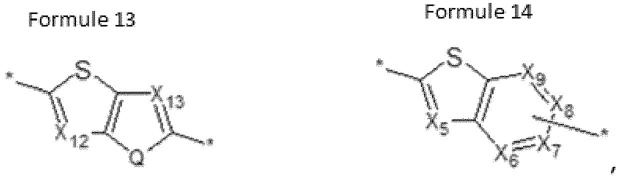 R₆ et R₇ étant choisis, à chaque fois indépendamment l'un de l'autre, dans le groupe constitué par H, alkyle et aryle.

4. Composé selon l'une quelconque des revendications précédentes, A1 étant choisi dans le groupe constitué par : 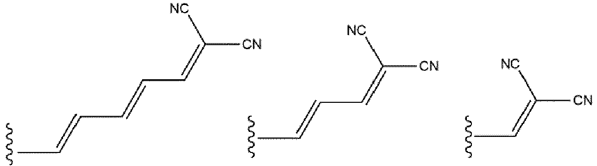 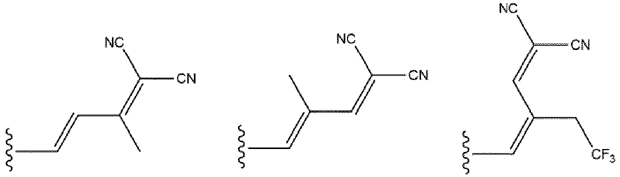 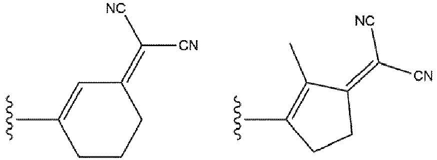 et
A2 étant choisi dans le groupe constitué par :

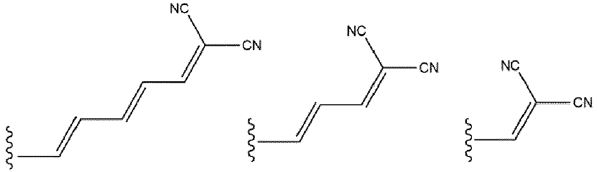

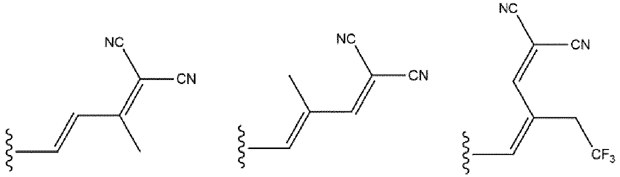

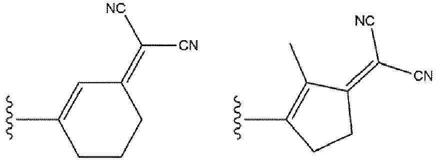

5. Composé selon l'une quelconque des revendications précédentes, les paramètres a, b, c représentant de préférence, à chaque fois indépendamment les uns des autres, 0 ou 1, à condition qu'au moins l'un des paramètres a, b, c représente 1, de préférence au moins deux des paramètres a, b, c représentant 1 ; ou le paramètre a représentant 1 ou 2, au moins un des paramètres b et c représentant 1 ou 2, de préférence un des paramètres b ou c représentant 1 ou 2, de préférence un des paramètres b ou c représentant 1, en particulier de préférence le paramètre c représentant 1 ou 2.

6. Composé selon l'une quelconque des revendications précédentes, le groupe M étant choisi dans le groupe constitué par : 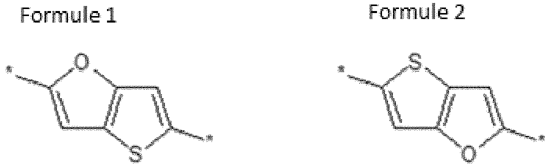 et
T1 étant choisi dans le groupe constitué par : 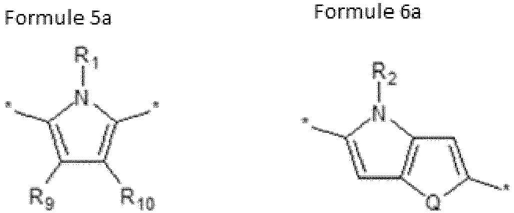 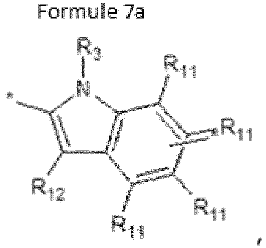 R₉ et R₁₀ étant choisis, à chaque fois indépendamment l'un de l'autre, dans le groupe constitué par H, halogène, de préférence F et Cl, CN, alkyle, alcényle, O-alkyle, S-alkyle, aryle, hétéroaryle ; R₉ et R₁₀ représentant de préférence, indépendamment l'un de l'autre, H ou alkyle ; R₁₁ et R₁₂ étant choisis, à chaque fois indépendamment l'un de l'autre, dans le groupe constitué par H, halogène, de préférence F et Cl, CN, alkyle, alcényle, O-alkyle, S-alkyle, aryle, hétéroaryle ; R₁₁ et R₁₂ représentant de préférence H.

7. Composé selon l'une quelconque des revendications précédentes, le composé présentant la formule générale II
A1-M-(T1)ₐ-(T3)_{c}-A2 (II),
de préférence, a et c représentant 1, a représentant 1 et c représentant 2 ou a représentant 2 et c représentant 1.

8. Composé selon l'une quelconque des revendications précédentes, le composé étant choisi dans le groupe constitué par :

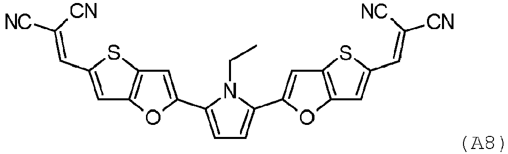

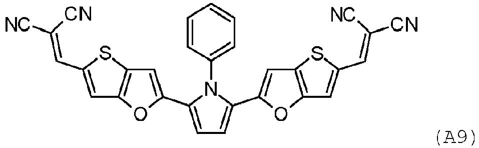

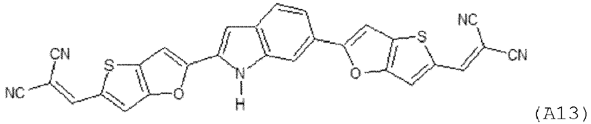

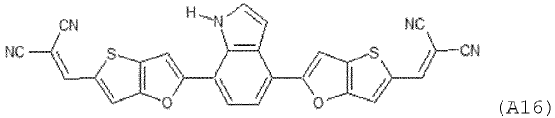

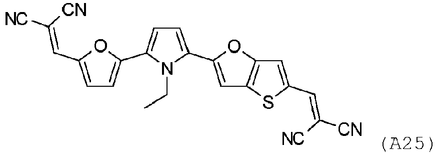

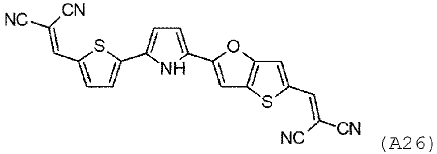

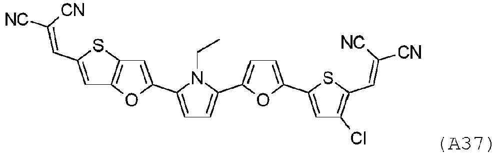

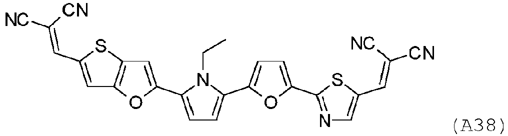

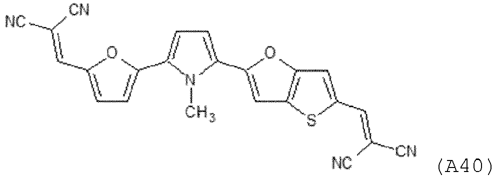

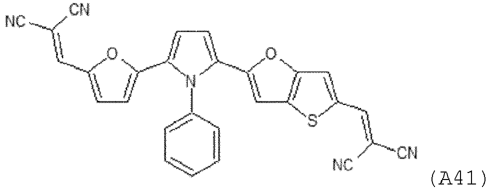

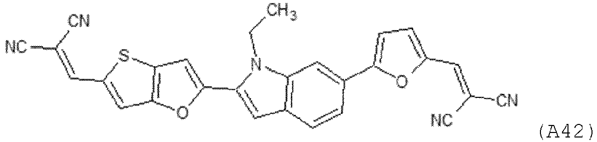

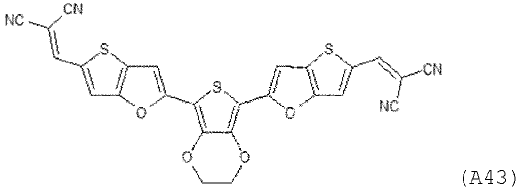

9. Utilisation d'au moins un composé selon l'une quelconque des revendications 1 à 8 dans un composant électronique organique, de préférence dans un élément organique photovoltaïque.

10. Composant électronique organique, comprenant une électrode, une contre-électrode et au moins une couche photoactive organique entre l'électrode et la contre-électrode, ladite au moins une couche photoactive organique présentant au moins un composé selon l'une quelconque des revendications 1 à 8.
